# EUROPEAN PATENT SPECIFICATION

(11) **EP 3 856 097 B1**
(45) Date of publication and mention of the grant of the patent: **24.01.2024**
(21) Application number: 19866325.4
(22) Date of filing: 26.09.2019
(51) Int. Cl.: A61F 5/32, A61F 5/34, A61G 7/057

(54) **SYSTEMS AND METHODS FOR CONTROLLING AND MONITORING INFLATABLE PERFUSION ENHANCEMENT APPARATUS FOR MITIGATING CONTACT PRESSURE**
SYSTEME UND VERFAHREN ZUR STEUERUNG UND ÜBERWACHUNG AUFBLASBARER PERFUSIONSVERBESSERUNGSVORRICHTUNGEN ZUR VERMINDERUNG DES KONTAKTDRUCKS
SYSTÈMES ET PROCÉDÉS DE COMMANDE ET DE SURVEILLANCE D'UN APPAREIL D'AMÉLIORATION DE PERFUSION GONFLABLE POUR ATTÉNUER UNE PRESSION DE CONTACT

(30) Priority: 26.09.2018 US 201862736758 P
(43) Date of publication of application: 04.08.2021
(73) Proprietor: TurnCare, Inc., Palo Alto, CA 94303 (US)
(72) Inventor: SQUITIERI, Rafael Paolo, Palo Alto, California 94303 (US); DEUTSCH, Robert Charles, Palo Alto, California 94303 (US); FRAZIER, Steven Bruce, Palo Alto, California 94303 (US)
(74) Representative: Ahmad, Sheikh Shakeel
(86) International application number: PCT/US2019/053246
(87) International publication number: WO 2020/069186

(56) References cited:
- EP-A2- 2 250 988
- US-A- 4 967 756
- US-A1- 2006 149 176
- US-A1- 2015 164 677
- US-A1- 2016 317 370
- US-A1- 2019 021 918
- US-B2- 9 901 491

## Description

### TECHNICAL FIELD

The present technology relates generally to apparatuses, systems, and methods for controlling and monitoring inflatable perfusion enhancement apparatuses that mitigate contact pressure applied to a human body by a support surface.

### BACKGROUND

Pressure injuries (sometimes referred to as "decubitus ulcers," "pressure ulcers," "pressure sores," or "bedsores") typically occur as a result of steady pressure applied in one location along a surface of the human body such as, for example, the sacrum. Pressure injuries are most common in individuals who are mobility-impaired or immobilized (e.g., in a wheelchair or a bed, or on an operating table) for prolonged periods of time. Oftentimes these individuals are older, malnourished, and/or incontinent, all factors that predispose the human body to pressure injury formation. Because these individuals are often not ambulatory, they may sit or lie for prolonged periods of time in the same position. Moreover, these individuals often are unable to reposition themselves to alleviate the pressure. Consequently, the pressure on the skin and soft tissue eventually causes ischemia or inadequate blood flow to the area, thereby resulting in breakdown of the skin and tissue damage. Pressure injuries can result in a superficial injury to the skin, or a deeper full-thickness ulcer that exposes underlying tissues and places the individual at risk for infection. The resulting infection may worsen, leading to sepsis, or even death in some cases.

There are various pressure technologies on the market for preventing pressure injuries. However, conventional alternating pressure technologies have many deficiencies, including the inability to control the spatial relationship between an individual and a support surface. Consequently, individuals using conventional alternating pressure technologies may still develop pressure injuries or suffer from related complications.

US 2016/317370 A1 describes a responsive cushion system including a pressure adjustment system and a cushion formed by an array of bladders coupled to the pressure adjustment system. At least two of the bladders are independent from one another such that that each can be independently pressurized and depressurized by the pressure adjustment system. A processor is coupled to the pressure adjustment system and a user interface, and the processor is operable to select a target group of one or more bladders according to data received via a communication interface. US 2015/164677 A1 describes systems and apparatuses for enhanced comfort through contact pressure reduction that prevent or otherwise mitigate pressure by actively orienting a patient over an anatomy-specific pressure-mitigating contact surface on which the patient rests. A pressure-mitigating contact portion of the contact surface includes a plurality of independently pressurized chambers configured in a specific geometric pattern that is designed to mitigate contact pressure between a support surface (e.g., bed or chair) and a specific anatomic region of a patient's body when the specific anatomic region of the patient's body is oriented over an epicenter of the geometric pattern.

### BRIEF DESCRIPTION OF THE DRAWINGS

Many aspects of the present disclosure can be better understood with reference to the following drawings. The components in the drawings are not necessarily to scale. Instead, emphasis is placed on clearly illustrating the principles of the present disclosure. Furthermore, components may be shown as transparent in certain views for the purpose of illustration, rather than to indicate that the component is necessarily transparent. Any headings provided herein are for convenience only.
Figures 1A and 1B are top and bottom views, respectively, of a pressure-mitigation apparatus configured in accordance with embodiments of the present technology.
Figures 2A and 2B are top and bottom views, respectively, of a pressure-mitigation apparatus configured in accordance with embodiments of the present technology.
Figure 3 is a top view of a pressure-mitigation apparatus configured in accordance with embodiments of the present technology.
Figure 4 is a partially schematic top view of a pressure-mitigation apparatus illustrating varied pressure distributions for avoiding ischemia for a mobility-impaired patient in accordance with embodiments of the present technology.
Figure 5A is a partially schematic side view of a pressure-mitigation apparatus for relieving pressure on a specific anatomical region by chamber deflation in accordance with embodiments of the present technology.
Figure 5B is a partially schematic side view of a pressure-mitigation apparatus for relieving pressure on a specific anatomical by chamber inflation in accordance with embodiments of the present technology.
Figures 6A-6C are isometric, front, and back views, respectively, of a controller device (for initiating chamber inflation and/or deflation for a pressure-mitigation device in accordance with embodiments of the present technology.
Figure 7 is a block diagram illustrating exemplary components of a controller configured in accordance with embodiments of the present technology.
Figure 8 is an isometric view of a manifold for controlling fluid flow to chambers of a pressure-mitigation apparatus in accordance with embodiments of the present technology.
Figure 9 is an electrical diagram illustrating piezoelectric valves of a manifold for separately controlling fluid flow along multiple channels in accordance with embodiments of the present technology.
Figure 10 is a flow diagram of a process for varying the pressure in chambers of a pressure-mitigation apparatus in accordance with embodiments of the present technology.
Figure 11 is a flow diagram of a process for establishing characteristics of a human body supported by a pressure-mitigation apparatus in accordance with embodiments of the present technology.
Figure 12 is a partially schematic side view of a pressure-mitigation system for orienting an individual over a pressure-mitigation apparatus in accordance with embodiments of the present technology.
Figure 13 is a block diagram illustrating a processing system in which at least some operations described herein can be implemented.

### DETAILED DESCRIPTION

Pressure injuries (also referred to a "pressure ulcers" or "ulcers") are localized regions of damage to the skin and/or the underlying tissue that result from contact pressure (or simply "pressure") on the corresponding anatomical region of the body. Pressure injuries often form over bony prominences, such as the skin and soft tissue overlying the sacrum, coccyx, heels, or hips. However, other sites (e.g., the elbows, knees, ankles, shoulders, abdomen, back, or cranium) may also be affected. Generally, pressure injuries develop when pressure is applied to blood vessels in soft tissue, which at least partially obstructs blood flow to the soft tissue (e.g., when the pressure exceeds the capillary filling pressure) and causes ischemia at the pressure site for an extended duration. Therefore, pressure injuries often occur in individuals who are mobility-impaired, immobilized, or sedentary for prolonged periods of times. Once a pressure injury forms, the healing process is typically slow. For example, when pressure is relieved from the site of the pressure injury, the body rushes blood (including proinflammatory mediators) to that region to perfuse the area. The sudden reperfusion of the damaged, previously ischemic region has been shown to cause an inflammatory response, brought on by the proinflammatory mediators, that can actually worsen the original pressure injury and prolongs recovery. Further, depending on the patient and the pressure injury, the proinflammatory mediators may spread through the blood stream beyond the site of the pressure injury to cause a systematic inflammatory response. This secondary inflammatory response caused by the proinflammatory mediators has been shown to exacerbate existing conditions or trigger additional ailments, thereby slowing recovery. Moreover, recovery time can be prolonged by numerous factors often associated with individuals prone to pressure injuries, such as old age, immobility, preexisting medical conditions (e.g., arteriosclerosis, diabetes, or infection), smoking, and/or medications (e.g., antiinflammatory drugs). Thus, preventing or reducing pressure injury formation (and reducing proinflammatory mediators) can enhance and expedite many treatment processes for individuals, especially those who are mobility-impaired during the course of treatment.

Introduced here, therefore, are systems and methods for controlling and monitoring inflatable perfusion enhancement apparatuses that mitigate contact pressure applied to a human body by a support surface. A controller device (also referred to as a "controller") can be fluidically coupled to a pressure-mitigation apparatus (also referred to as a "pressure-mitigation device" or a "pressure-mitigation pad") that includes a series of selectively inflatable chambers (also referred to as "cells"). When the pressure-mitigation apparatus is placed between a human body and a support surface (also referred to as a "contact surface"), the controller device can continuously and intelligently circulate air through the pressure mitigation apparatus. The controller device causes one or more chambers of the pressure-mitigation device to selectively inflate, deflate, or any combination thereof.

By controllably varying the pressure in the series of chambers, the controller device can move the main point of pressure applied by the support surface to various different regions across the human body. For example, following deployment of the pressure-mitigation apparatus, the controller device can move the main point(s) of pressure applied by the support surface amongst a plurality of predetermined locations by sequentially varying the level of inflation of and, therefore, pressure in different predetermined subsets of inflatable chambers. In some embodiments, the controller controls pressure beneath specific anatomic locations of the patient for specific durations in order to move pressure points around the anatomy in a precise manner such that specific portions of the anatomy (e.g., tissue adjacent bony prominences) have minimal pressure applied for predetermined periods of time. This continuous or intermittent relocation of the pressure point(s) avoids vascular compression for sustained periods of time and, therefore, inhibits ischemia and ultimately reduces the incidence of pressure injuries.

In addition, the controller device can provide various different features and functions that provide for and enhance dynamic control of the pressure-mitigation device. The controller device is configured to auto-detect the type of pressure-mitigation device attached thereto and configure the pressure mitigation inflation-deflation protocol for that type of device.

The controller device can also provide alerts to the patient, caregivers, and others related to the functionality of the pressure-mitigation device and patient monitoring (e.g., improper usage, compliance to treatment protocol). In some embodiments, for example, the controller device can also detect patient motion on the pressure mitigation device by remotely monitoring the pressures within the air chambers, and then use this information to determine information in real-time regarding patient movement and patient location on the device.

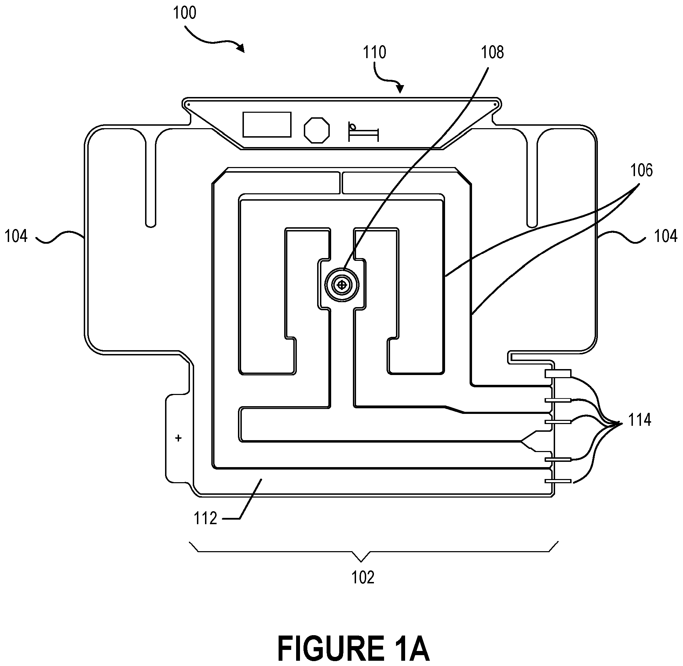

Specific details of several embodiments of the present technology are described herein with reference to Figures 1A-14. Although many of the embodiments are described herein with respect to systems, apparatuses, and methods for controlling inflatable perfusion enhancement apparatuses and associated systems and methods for alleviating the pressure applied to a human body (e.g., a patient, individual, or subject) in a certain position (e.g., the supine position) by a support surface (e.g., a mattress), other embodiments in addition to those described herein are within the scope of the present technology. For example, at least some embodiments of the present technology may be useful for alleviating the pressure applied to a human body in a sitting position. In such embodiments, the chambers of the pressure-mitigation apparatus may be different sizes, in different arrangements, and/or otherwise differ from the chambers of pressure-mitigation apparatuses for patients oriented in a supine position. Additionally or alternatively, the chambers of the pressure-mitigation apparatus may be inflated in a different order, with different pressures, for different durations, and/or otherwise have a different inflation pattern than those of pressure-mitigation apparatuses for patients oriented in a supine position.

It should be noted that other embodiments in addition to those disclosed herein are within the scope of the present technology, if they fall under the scope defined by the appended claims defining the invention. For example, components, configurations, and/or procedures shown or described with respect to one embodiment can be combined with or replace the components, configurations, and/or procedures described in other embodiments. Further, embodiments of the present technology can have different components, configurations, and/or procedures than those shown or described herein. Moreover, a person of ordinary skill in the art will understand that embodiments of the present technology can have configurations, components, and/or procedures in addition to those shown or described herein, and that these and other embodiments can be without several of the configurations, components, and/or procedures shown or described herein without deviating from the present technology.

### Selected Embodiments of Pressure-Mitigation Apparatuses

A pressure-mitigation apparatus includes a plurality of chambers or compartments that can be individually controlled to vary the pressure in each chamber and/or a subset of the chambers. When placed between a human body and a support surface, the pressure-mitigation apparatus can vary the pressure on an anatomical region by controllably inflating one or more chambers, deflating one or more chambers, or any combination thereof. Several examples of pressure-mitigation apparatuses are described below with respect to Figures 1A-3. Unless otherwise noted, any features described with respect to one embodiment are equally applicable to the other embodiments. Some features have only been described with respect to a single embodiment of the pressure-mitigation apparatus for the purpose of simplifying the present disclosure.

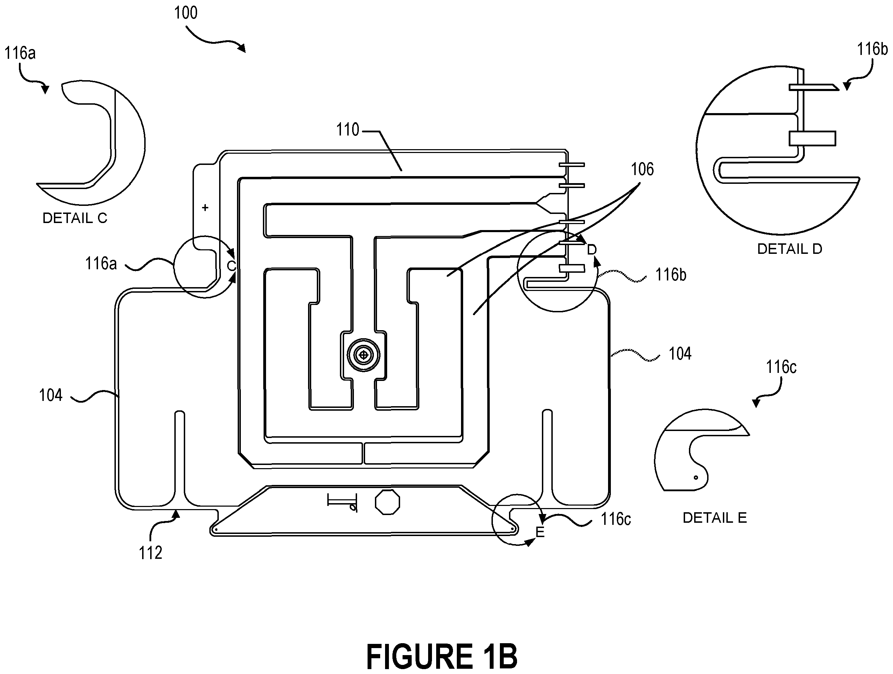

Figures 1A and 1B are top and bottom views, respectively, of a pressure-mitigation apparatus 100 for relieving pressure on a specific anatomical region applied by an elongated support surface in accordance with embodiments of the present technology. The pressure-mitigation apparatus 100 can be used in conjunction with elongated support surfaces, such as mattresses, stretchers, operating tables, and procedure tables. In some embodiments the pressure-mitigation apparatus 100 is secured to a support surface using an attachment apparatus, while in other embodiments the pressure-mitigation apparatus 100 is placed in direct contact with the support surface (i.e., without any attachment apparatus therebetween).

As shown in Figure 1A, the pressure-mitigation apparatus 100 can include a central portion 102 (also referred to as a "contact portion") positioned alongside at least one side support 104. Here, a pair of side supports 104 are arranged on opposing sides of the central portion 102. However, some embodiments of the pressure-mitigation apparatus 100 do not include any side supports. For example, the side support(s) 104 may be omitted when the individual is medically immobilized (e.g., under anesthesia, in a medically induced coma, etc.) and/or physically restrained by the underlying support surface (e.g., by rails along the side of a bed, armrests along the side of a chair) and/or other structures (e.g., physical restraints holding down the patient, casts, etc.).

The pressure-mitigation apparatus 100 includes a series of chambers 106 (also referred to as "cells") whose pressure can be individually varied. In some embodiments, the series of chambers 106 are arranged in a geometric pattern designed to relieve pressure on one or more specific anatomical regions of a human body. As noted above, when placed between the human body and a support surface, the pressure-mitigation apparatus 100 can vary the pressure on the specific anatomical region(s) by controllably inflating chamber(s), deflating chamber(s), or any combination thereof.

In some embodiments, the geometric pattern is designed to mitigate pressure on a specific anatomical region when the specific anatomical region is oriented over a target region 108 of the geometric pattern. As shown in Figures 1A and 1B, the target region 108 may represent a central point or portion of the pressure-mitigation apparatus 100 to appropriately position the person's anatomy with respect to the pressure-mitigation apparatus 100. For example, the target region 108 may correspond to an epicenter of the geometric pattern. However, the target region 108 may not necessarily be the central point of the pressure-mitigation apparatus 100, particularly if the pressure-mitigation apparatus 100 is not symmetric. The target region 108 may be marked so that an individual (e.g., a physician, nurse, caregiver, or the patient himself or herself) can readily align the target region 108 with a corresponding anatomical region of the human body to be positioned thereon.

The pressure-mitigation apparatus 100 can include a first portion 110 (also referred to as a "first layer" or a "bottom layer") designed to face a support surface and a second portion 112 (also referred to as a "second layer" or a "top layer") designed to face the human body supported by the support surface. In some embodiments the first portion 110 is directly adjacent to the support surface, while in other embodiments the first portion 110 is directly adjacent to an attachment apparatus designed to help secure the pressure-mitigation apparatus 100 to the support surface. The pressure-mitigation apparatus 100 may be constructed of a variety of materials, and the material(s) used in the construction of each component of the pressure-mitigation apparatus 100 may be chosen based on the nature of the body contact, if any, to be experienced by the component. For example, because the second portion 112 will often be in direct contact with the skin, it may be comprised of a soft fabric or a breathable fabric (e.g., comprised of moisture-wicking materials or quick-drying materials, or having perforations). In some embodiments, an impervious lining (e.g., comprised of polyurethane) is secured to the inside of the second portion 112 to inhibit fluid (e.g., sweat) from entering the series of chambers 106. As another example, if the pressure-mitigation apparatus 100 is designed for deployment beneath a cover (e.g., a bed sheet), then the second portion 112 may be comprised of a liquid-impervious, flexible material, such as polyurethane, polypropylene, silicone, or rubber. The first portion 110 may also be comprised of a liquid-impervious, flexible material.

The series of chambers 106 may be formed via interconnections between the first and second portions 110, 112 (e.g., either directly or via one or more intermediary layers). In the embodiment illustrated in Figures 1A and 1B, the pressure-mitigation apparatus 100 includes an "M-shaped" chamber intertwined with two "C-shaped" chambers that face one another. Such an arrangement has been shown to effectively mitigate the pressure applied to the sacral region of a human body in the supine position by a support surface when the pressure in these chambers is alternated. A pressure-mitigation apparatus may have another arrangement of chambers if the pressure-mitigation apparatus is designed for an anatomical region other than the sacral region, or if the pressure-mitigation apparatus is to be used to support a human body in a non-supine position (e.g., a sitting position). Generally, the geometric pattern of the chambers 106 is designed based on the internal anatomy (e.g., the muscles, bones, and vasculature) of a specific anatomical region on which pressure is to be relieved.

The person using the pressure-mitigation apparatus 100 and/or the caregiver (e.g., a nurse, physician, etc.) will often be responsible for actively orienting the anatomical region of the patient lengthwise over the target region 108 of the geometric pattern. However, the side support(s) 104 may actively orient or guide the specific anatomical region of the human body laterally over the target region 108 of the geometric pattern. In some embodiments the side support(s) 104 are inflatable, while in other embodiments the side support(s) 104 are permanent structures that protrude from one or both lateral sides of the pressure-mitigation device 100. For example, at least a portion of each side support may be stuffed with cotton, latex, polyurethane foam, or any combination thereof.

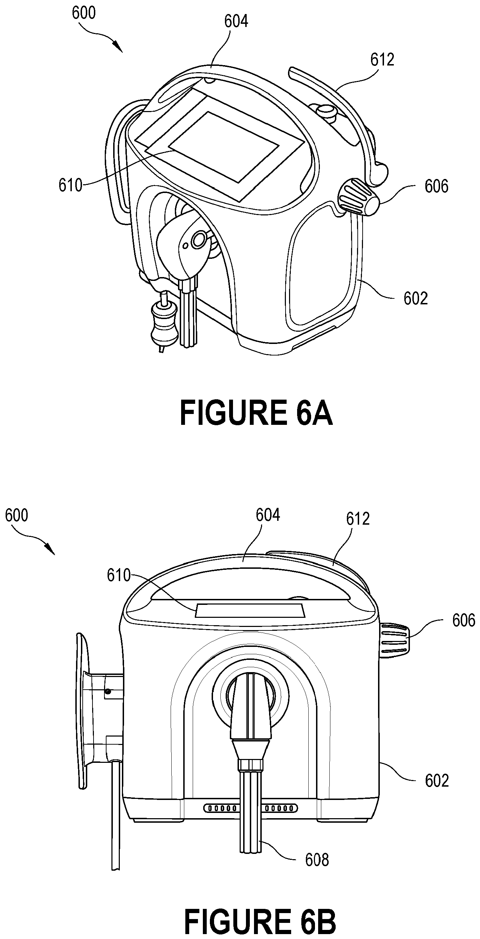

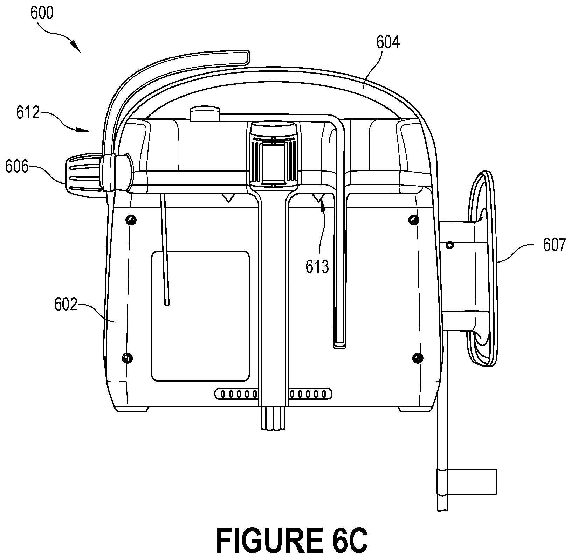

As further described below with respect to Figures 6A and 6B, a controller device can separately control the pressure in each chamber (as well as the side supports 104, if included) by providing a discrete airflow via one or more corresponding valves 114. In some embodiments, the valves 114 are permanently secured to the pressure-mitigation apparatus 100 and designed to interface with tubing that can be readily detached (e.g., for easier transport, storage, etc.). Here, the pressure-mitigation apparatus 100 includes five valves 114. Three valves are fluidically coupled to the series of chambers 106, and two valves are fluidically coupled to the side supports 104. In other embodiments, the pressure-mitigation apparatus 100 includes more than five valves 114 and/or less than five valves 114.

In some embodiments, the pressure-mitigation apparatus 100 includes one or more structural feature(s) 116a-c that enhance securement of the pressure-mitigation apparatus 100 to a support surface and/or an attachment apparatus. As illustrated in Figure 1B, for example, the pressure-mitigation apparatus 100 can include three design feature(s) 116a-c, each of which can be aligned with a corresponding structural feature that is accessible along the support surface or the attachment apparatus. For example, each design feature 116a-c may be designed to at least partially envelope a structural feature that protrudes upward. The design feature(s) 116a-c may also facilitate proper alignment of the pressure-mitigation apparatus 100 with the support surface or the attachment apparatus.

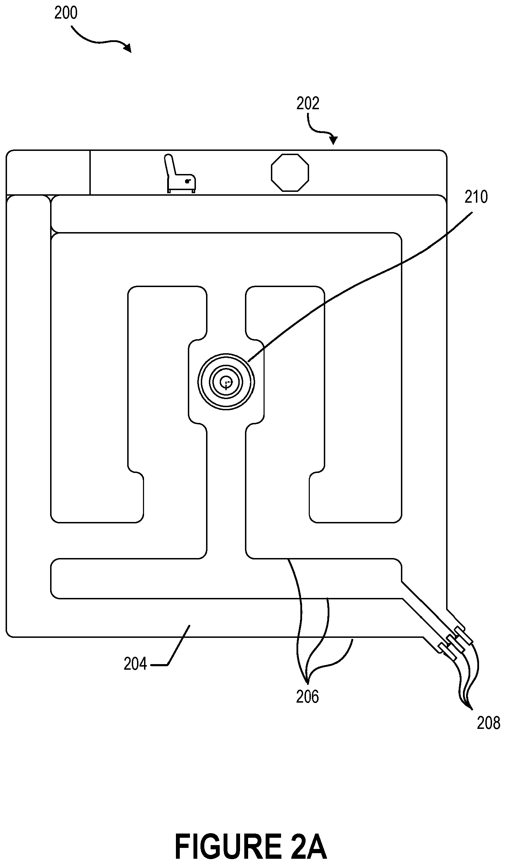

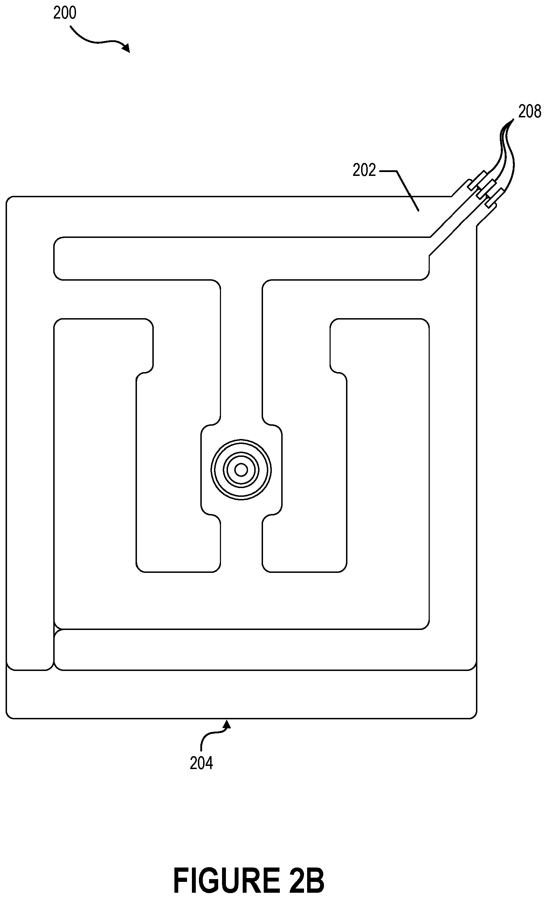

Figures 2A and 2B are top and bottom views, respectively, of a pressure-mitigation apparatus 200 configured in accordance with embodiments of the present technology. The pressure-mitigation apparatus 200 is generally used in conjunction with nonelongated support surfaces that support individuals in a seated or partially erect position, such as chairs (e.g., office chairs, examination chairs, recliners, and wheelchairs) and the seats included in vehicles and airplanes. As such, the pressure-mitigation apparatus 200 will often be positioned atop support surfaces that have side supports integrated into the support itself (e.g., the side arms of a recliner or wheelchair). In some embodiments the pressure-mitigation apparatus 200 is secured to a support surface using an attachment apparatus, while in other embodiments the attachment apparatus is omitted such that the pressure-mitigation apparatus 200 directly contacts the underlying support surface.

The pressure-mitigation apparatus 200 can include various features generally similar to the features of the pressure-mitigation device 100 described above with respect to Figures 1A and 1B. For example, the pressure-mitigation apparatus 200 may include a first portion 202 (also referred to as a "first layer" or a "bottom layer") designed to face the support surface, a second portion 204 (also referred to as a "second layer" or a "top layer") designed to face the human body supported by the support surface, and a plurality of chambers 206 formed via interconnections between the first and second portions 202, 204. In this embodiment, the pressure-mitigation apparatus 200 includes an "M-shaped" chamber 206 intertwined with a backward "J-shaped" chamber 206 and a backward "C-shaped" chamber 206. The alternating inflation/deflation of such an arrangement of chambers 206 has been shown to effectively mitigate the pressure applied by a support surface to the sacral region when the human body is in a seated position.

The individual inflation/deflation of these chambers 206 can be performed in a predetermined pattern and to predetermined pressure levels. In some embodiments, for example, the individual chambers 206 can be inflated to higher pressure levels than the chambers 206 of the pressure-mitigation apparatus 100 described with respect to Figures 1A and 1B because the human body supported by the pressure-mitigation apparatus 200 is in a seated position, thereby applying more pressure on the pressure-mitigation apparatus 200 than if the human body were supine or prone. Further, unlike the pressure mitigation device 100 of Figures 1A and 1B, the pressure-mitigation apparatus 200 of Figures 2A and 2B does not include side supports. As noted above, side supports may be omitted when the structure on which the individual is seated or reclined already provides components that laterally center the individual (e.g., rails along the side of a bed, armrests along the side of a chair), as is often the case with nonelongated support surfaces.

As further described below with respect to Figures 6A and 6B, a controller device can control the pressure in each chamber 206 by providing a discrete airflow via one or more corresponding valves 208. Here, the pressure-mitigation apparatus 200 includes three valves 208, and each of the three valves 208 corresponds to a single chamber 206. In other embodiments, the pressure-mitigation apparatus 200 may include one valve, two valves, or more than three valves 208, and each valve 208 can be associated with a specific chamber for individually controlled inflation and/or deflation of that chamber 206. In these and other embodiments, a single valve 208 can be fluidically coupled to two or more chambers 206. In these and other embodiments, a single chamber 206 can be in fluid communication with two or more valves 208 (e.g., one valve for inflation and another valve for deflation).

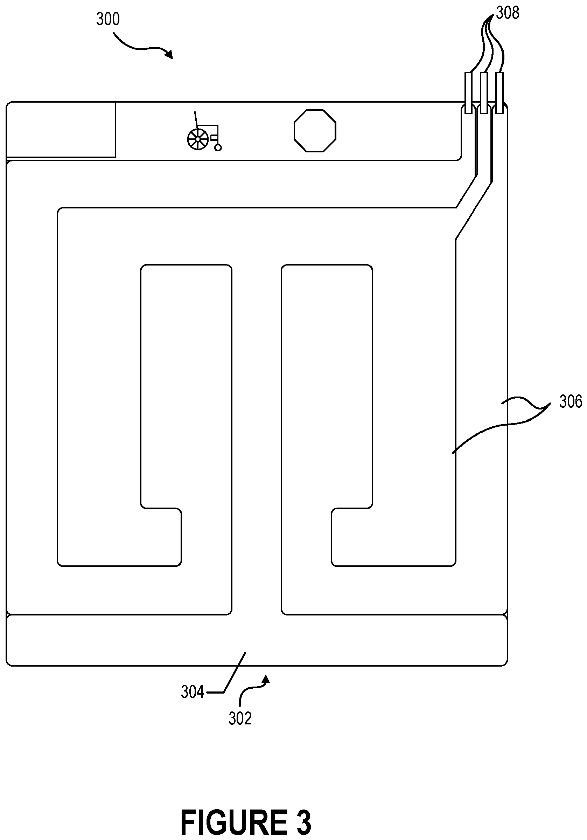

Figure 3 is a top view of a pressure-mitigation apparatus 300 for relieving pressure on a specific anatomical region applied by a wheelchair in accordance with embodiments of the present technology. The pressure-mitigation apparatus 300 can include various features generally similar to the features of the pressure-mitigation apparatus 200 of Figures 2A and 2B and the pressure-mitigation apparatus 100 of Figures 1A and 1B described above. For example, the pressure-mitigation apparatus 300 can include a first portion 302 (also referred to as a "first layer" or a "bottom layer") designed to face the seat of the wheelchair (i.e., the support surface), a second portion 304 (also referred to as a "second layer" or a "top layer") designed to face the human body supported by the seat of the wheelchair, a plurality of chambers 306 formed by interconnections between the first and second portions 302, 304, and a plurality of valves 308 that control the flow of fluid into and/or out of the chambers 306. In some embodiments the first portion 302 is directly adjacent to the seat of the wheelchair, while in other embodiments the first portion 302 is directly adjacent to an attachment apparatus. As shown in Figure 3, the pressure-mitigation apparatus 300 may include an "M-shaped" chamber 306 intertwined with a "U-shaped" chamber 306 and a "C-shaped" chamber 306, which are inflated and deflated in accordance with a predetermined pattern to mitigate the pressure applied to the sacral region of a human body in a sitting position on the seat of a wheelchair.

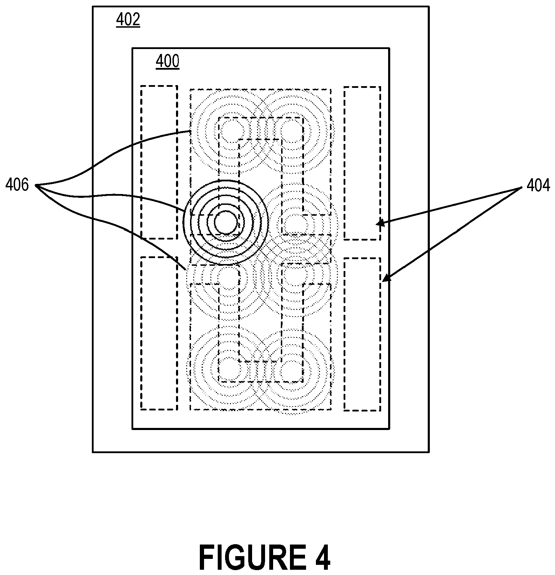

Figure 4 is a partially schematic top view of a pressure-mitigation apparatus 400 illustrating varied pressure distributions for avoiding ischemia for a mobility-impaired patient in accordance with embodiments of the present technology. As discussed above, when a human body is supported by a contact surface 402 for an extended duration, pressure injuries may form in tissue overlaying bony prominences, such as the skin overlying the sacrum, coccyx, heels, or hips. These bony prominences often represent the location or locations at which the most pressure is applied by the contact surface 402 and, therefore, may be referred to as the "main pressure point(s)" along the surface of the human body. To prevent the formation of pressure injuries, healthy individuals periodically make minor positional adjustments (also known as "micro-adjustments") to shift the location of the main pressure point. However, individuals having impaired mobility often cannot make these micro-adjustments by themselves. Mobility impairment may be due to physical injury (e.g., a traumatic injury or a progressive injury), movement limitations (e.g., within a vehicle, on an aircraft, or in restraints), medical procedures (e.g., those requiring anesthesia), and/or other conditions that limit an individual's natural movement. For these mobility-impaired individuals, the pressure-mitigation apparatus 400 can be used to shift the location of the main pressure point(s) on their behalf. That is, the pressure mitigation apparatus 400 can create moving pressure gradients to avoid sustained, localized vascular compression and enhance tissue perfusion.

As shown in Figure 4, the pressure-mitigation apparatus 400 can include a series of chambers 404 (also referred to as "cells") whose pressure can be individually varied. The chambers 404 may be formed by interconnections between a first or bottom layer and a second or top layer of the pressure-mitigation apparatus 400. The top layer may be comprised of a first material (e.g., an air-permeable, non-irritating material) configured for direct contact with a human body, while the bottom layer may be comprised of a second material (e.g., a non-air-permeable, gripping material) configured for direct contact with the contact surface 402 or an attachment apparatus. In these and other embodiments, the top layer and/or the bottom layer can be comprised of more than one material, such as a coated fabric or a stack of interconnected materials.

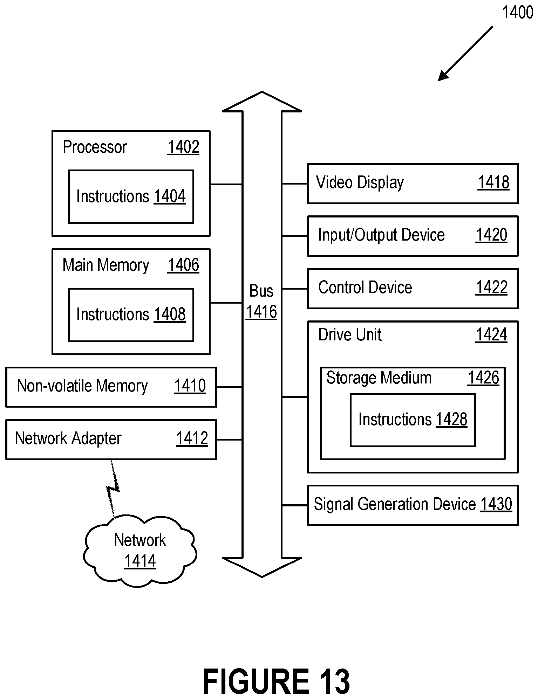

A pump, such as the pressure device 1314 described below with respect to Figure 13, can be fluidically coupled to each chamber 404 (e.g., via a corresponding inlet valve), while a controller, such as the controller 1312 described below with respect to Figure 13, can control the flow of fluid (e.g., air) generated by the pump into each chamber 404 on an individual basis in accordance with a predetermined pattern. As further described below, the pump and controller can operate the series of chambers 404 in several different ways. In some embodiments, the chambers 404 have a naturally deflated state, and the controller causes the pump to inflate at least one of the chambers 404 to shift the main pressure point along the anatomy of the user. For example, the pump may inflate at least one of the chambers 404 located directly beneath an anatomical region to momentarily apply contact pressure to that anatomical region and relieve the contact pressure on the surrounding anatomical regions adjacent to the deflated chamber(s) 404. In these and other implementations, the controller may cause the pump to inflate two or more chambers 404 adjacent to an anatomical region to create an open space or void beneath the anatomical region to shift the main pressure point at least momentarily away from the anatomical region. In other embodiments, the chambers 404 have a naturally inflated state, and the controller causes the pump to deflate at least one of the chambers 404 to shift the main pressure point along the anatomy of the user. For example, the pump may be configured to deflate at least one of the chambers 404 located directly beneath an anatomical region, thereby forming a void beneath the anatomical region to momentarily relieve the contact pressure on the anatomical region. Whether configured in a naturally deflated state or a naturally inflated state, the continuous or intermittent alteration of the inflation levels of the individual chambers 404 moves the location of the main pressure point across different portions of the human body. As shown in Figure 4, for example, inflating and/or deflating the chambers 404 creates temporary contact regions 406 that move across the pressure-mitigation apparatus 400 in a predetermined pattern, and thereby change the location of the main pressure point(s) on the human body for finite intervals of time. Thus, the pressure-mitigation apparatus 400 can simulate the micro-adjustments made by mobile individuals to relieve stagnant pressure application caused by the contact surface 402.

As noted above, the series of chambers 404 may be arranged in an anatomy-specific pattern so that when the pressure within one or more individual chambers is altered, the contact pressure on a specific anatomical region of the human body is relieved (e.g., by shifting the main pressure point elsewhere). As shown in Figure 4, for example, the main pressure point can be moved between eight different locations corresponding to the eight temporary contact regions 406. In some embodiments the main pressure point shifts between these locations in a predictable manner (e.g., in a clockwise or counter-clockwise pattern), while in other embodiments the main pressure point shifts between these locations in an unpredictable manner (e.g., in accordance with a random pattern, a semi-random pattern, and/or detected pressure levels). Those skilled in the art will recognize that the quantity and position of these temporary contact regions 406 may vary based on the arrangement of the series of chambers 404, the anatomical region supported by the pressure-mitigation apparatus 400, the characteristics of the human body supported by the pressure mitigation apparatus 400, and/or the condition of the user (e.g., whether the user is completely immobilized, partially immobilized, etc.).

In some embodiments, the pressure-mitigation apparatus 400 does not include side supports because the condition of the user (also referred to as a "patient") may not benefit from the positioning provided by the side supports. For example, side supports can be omitted when the patient is medically immobilized (e.g., under anesthesia, in a medically induced coma, etc.) and/or physically restrained by the underlying support surface (e.g., rails along the side of a bed, arm rests on the side of a chair) and/or other structures (e.g., physically restraints holding down the patient, casts, etc.).

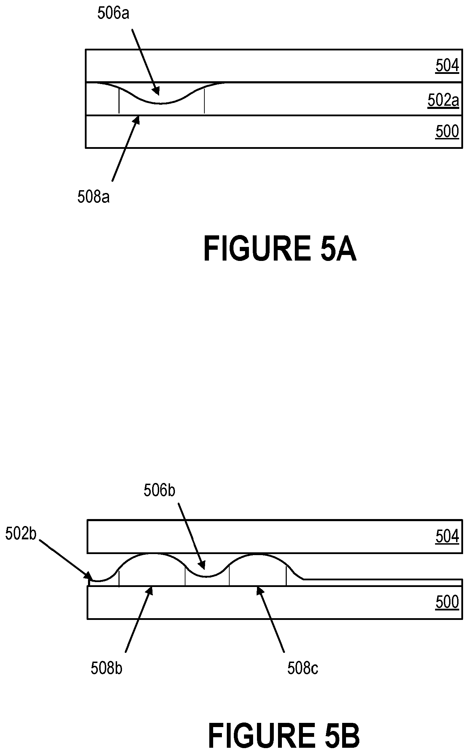

Figure 5A is a partially schematic side view of a pressure-mitigation apparatus 502a for relieving pressure on a specific anatomical region by chamber deflation in accordance with embodiments of the present technology. The pressure-mitigation apparatus 502a can be positioned between a contact surface 500 (e.g., a bed, table, or chair) and a human body 504 and, to relieve pressure on a specific anatomical region of the human body 504, at least one chamber 508a of a plurality of chambers (referred to collectively as "chambers 508") proximate to the specific anatomical region at least partially deflates to create an open region or void 506a beneath the specific anatomical region. In such embodiments, the remaining chambers 508 may remain inflated. Thus, the pressure-mitigation apparatus 502a may sequentially deflate chambers 508 (or arrangements of multiple chambers) to relieve the contact pressure applied to the human body 504 by the contact surface 500.

Figure 5B is a partially schematic side view of a pressure-mitigation apparatus 502b for relieving pressure on a specific anatomical by chamber inflation in accordance with embodiments of the present technology. For example, to relieve pressure at a specific anatomical region of the human body 504, the pressure-mitigation apparatus 502b can inflate two chambers 508b and 508c disposed directly adjacent to the specific anatomical region to create a void 506b beneath the specific anatomical region. In such embodiments, the remaining chambers may remain at least partially deflated. Thus, the pressure-mitigation apparatus 502b may sequentially inflate a chamber (or arrangements of multiple chambers) to relieve the contact pressure applied to the human body 504 by the contact surface 500.

The pressure-mitigation apparatuses 502a and 502b of Figures 5A and 5B are shown to be in direct contact with the contact surface 500. However, in some embodiments, an attachment apparatus is positioned between the pressure-mitigation apparatuses 502a and 502b and the contact surface 500.

In some embodiments, the pressure-mitigation apparatuses 502a and 502b of Figures 5A and 5B can have the same configuration of chambers 508, and can operate in both a normally inflated state (described with respect to Figure 5A) and a normally deflated state (described with respect to Figure 5B) based on the selection of the operator (e.g., a medical professional or the user). For example, the operator can use a controller to select a normally deflated mode such that the pressure-mitigation device operates as described with respect to Figure 5A, and then change the mode of operation to a normally inflated mode such that the pressure-mitigation device operates as described with respect to Figure 5B. Thus, the pressure-mitigation apparatuses disclosed herein can shift the location of the main pressure point by controllably inflating the chambers, controllably deflating the chambers, or a combination thereof.

### Selected Embodiments of Controller Devices

Figures 6A-6C are isometric, front, and back views, respectively, of a controller device 600 (also referred to as "the controller 600") for initiating chamber inflation and/or deflation of a pressure-mitigation device in accordance with embodiments of the present technology. For example, the controller 600 can be coupled to the pressure-mitigation apparatuses 100, 200, 300 described above with respect to Figures 1A-3 to control the pressure within the chambers 106, 206, 306. The controller 600 can manage the pressure in each chamber of a pressure-mitigation apparatus by controllably driving one or more pumps. In some embodiments, a single pump is fluidically connected to all the chambers such that the pump is responsible for directing fluid flow to and/or from multiple chambers. In other embodiments, the controller 600 is coupled to two or more pumps, each of which can be fluidically coupled to a single chamber to drive inflation/deflation of that chamber. In other embodiments, the controller 600 is coupled to at least one pump that is fluidically coupled to two or more chambers and/or at least one pump that is fluidically coupled to a single chamber. The pump(s) can reside within the same housing as the controller itself such that the system is easily transportable. Alternatively, the one or more pumps may reside in a housing separate from the controller.

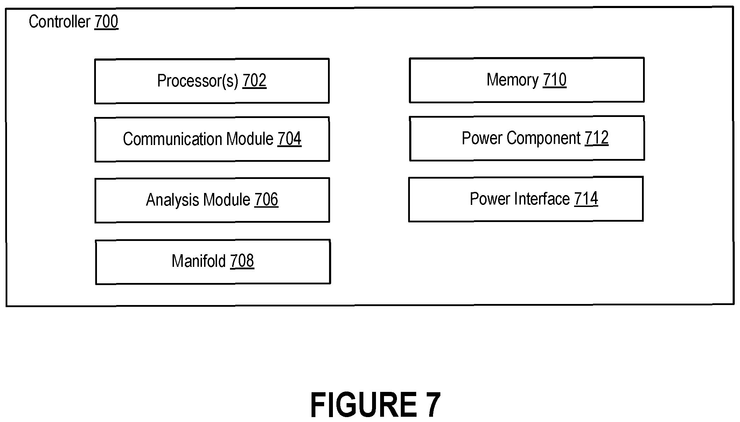

As shown in Figures 6A-6C, the controller 600 can include a housing 602 in which internal components (e.g. those described below with respect to Figure 7) reside and a handle 604 connected to the housing 602. In some embodiments the handle 604 is fixedly secured to the housing 602 in a predetermined orientation, while in other embodiments the handle 604 is pivotably secured to the housing 602. For example, the handle 604 may be configured to rotate about a hinge connected to the housing between multiple positions. The hinge may be one of a pair of hinges connected to the housing 602 along opposing lateral sides. In some embodiments, the controller device 600 can include cord retention mechanism 607 that is attached to or integrated with the housing 606. Cords (e.g., electrical cords), tubes, and/or other elongated structures associated with the system can be wrapped around or otherwise supported by the cord retention mechanism 607. Thus, the cord retention mechanism 607 can provide strain relief and retention of the power cord and, in certain embodiments, can also provide a flexible flange that retains the power cord plug.

As further shown in Figures 6A-6C, the controller 600 may include a connection mechanism 612 that allows the housing 602 to be securely, yet releasably, attached to a structure (e.g., of a mobile cart, bedframe, rail, table). In the illustrated embodiment, the connection mechanism 612 is a mounting hook that allows for single hand operation and is adjustable to allow for attachment to mounting surfaces with various thicknesses. In some embodiments, the controller device 600 can include an integrated intravenous (IV) pole clamp 613 that eases attachment of the controller device 600 to IV poles. The IV pole clamp may provide for quick activation and can be self-centering with the use of a single activation mechanism (e.g., knob or button).

In some embodiments, the housing 602 includes one or more mechanical input components 606 for providing instructions to the controller 600. The input components 606 may include one or more knobs (e.g., as shown in Figures 6A-6C), dials, buttons, levers, and/or other actuation mechanisms. An operator can interact with the one or more input components 606 to alter airflow provided to the pressure-mitigation apparatus, discharge air from the pressure-mitigation apparatus, or disconnect the controller 600 from the pressure-mitigation apparatus (e.g., by disconnecting the controller 600 from tubing connected between the controller 600 and the pressure-mitigation apparatus).

As further described below, the controller 600 can be configured to inflate and/or deflate the individual chambers of a pressure-mitigation apparatus in a predetermined pattern. In some embodiments at least one pressure device (e.g., an air pump) resides in the housing 602 of the controller 600, while in other embodiments the controller 600 is fluidically connected to at least one pressure device. For example, the housing 602 includes a first fluid interface through which fluid is received from pressure device(s) and a second fluid interface through which fluid is directed to the pressure-mitigation apparatus. Multi-channel tubing may be connected to one or both of these fluid interfaces. For example, multi-channel tubing may be connected between the first fluid interface of the controller 600 and multiple pressure devices. As another example, multi-channel tubing may be connected between the second fluid interface of the controller 600 and multiple valves of the pressure-mitigation apparatus. Here, the controller 600 includes a fluid interface 608 designed to interface with a multi-channel tubing. In some embodiments the multi-channel tubing permits unidirectional fluid flow, while in other embodiments the multi-channel tubing permits bidirectional fluid flow. Thus, fluid returning from the pressure-mitigation apparatus (e.g., as part of a discharge process) may travel back to the controller 600 through the second fluid interface. By controlling the exhaust of fluid returning from the pressure-mitigation apparatus, the controller 600 can actively manage noise created during use.

By monitoring the connection with the fluid interface 608, the controller 600 is able to detect which type of pressure-mitigation apparatus has been connected. Each type of pressure-mitigation apparatus may include a different type of connector. For example, the pressure-mitigation apparatus designed for elongated support surfaces (e.g., pressure-mitigation apparatus 100 of Figures 1A-B) may include a first arrangement of magnets in its connector, while the pressure-mitigation apparatus designed for non-elongated support surfaces (e.g., pressure-mitigation apparatus of Figures 2A-B) may include a second arrangement of magnets in its connector. The controller 600 includes one or more sensor(s) arranged near the fluid interface 608 for detecting whether magnets are located within a specified proximity. The controller 600 may automatically determine, based on which magnets have been detected by the sensor(s), which type of pressure-mitigation apparatus is connected. For example, pressure-mitigation devices may have different geometries, layouts, and/or dimensions suitable for various different patient positions (e.g., supine, prone, sitting), the support surface on which they are designed to reside (e.g., wheelchair, bed, recliner, surgical table), and/or patient characteristics (e.g., indication, size), and the controller can be configured to automatically detect the type of pressure-mitigation device connected thereto. In some embodiments, the automatic detection is performed using other suitable identification mechanisms, such as the controller device 600 reading an RFID tag or bar code on the pressure-mitigation device. As further described below, the controller 600 is configured to dynamically alter the pattern for inflating chambers based on which type of pressure-mitigation apparatus is connected.

The controller 600 may also include a display 610 for displaying information related to the pressure-mitigation apparatus, the pattern of inflations/deflations, the patient, etc. For example, the display 610 may present an interface that specifies which type of pressure-mitigation apparatus (e.g., pressure-mitigation apparatus 100, 200, 300 of Figures 1A-3) is connected to the controller 600. Other display technologies could also be used to convey information to an operator of the controller 600. In some embodiments, the controller 600 includes a series of lights (e.g., light-emitting diodes) that are representative of different statuses to provide visual alerts to the operator or user. For example, a status light may provide a green visual indication if the controller 600 is presently providing therapy, a yellow visual indication if the controller 600 has been paused (i.e., is in a pause mode), a red visual indication if the controller 600 has experienced an issue (e.g., noncompliance of patient, patient not detected on device) or requires maintenance (i.e., is in an alert mode), etc. These visual indications may dim upon the conclusion of a specified period of time or upon determining that the status has changed (e.g., the pause mode is no longer active).

In some embodiments, the controller device 600 can also include a quick or rapid deflate function that allows a clinician to rapidly deflate all or a portion (e.g., the side chambers) of the pressure-mitigation device. This is a software solution provided by the controller device 600 and activated via the display 610 (e.g., when configured as a user interface with touchscreen buttons) and/or tactile actuators (e.g., buttons) on the device. This rapid deflation, in particular the deflation of the side pillows, is expected to be beneficial to clinicians when there is a need for quick access to the patient, such as to provide CPR.

Figure 7 is a block diagram illustrating components of a controller 700 in accordance with embodiments of the present technology. The controller 700 includes a processor and a memory and it can include more processors 702, a communication module 704, an analysis module 706, a manifold 708, and/or a power component 712 that is electrically coupled to a power interface 714. These components may reside within a housing (also referred to as a "structural body"), such as the controller device housing 602 described above with respect to Figures 6A-6C. In some embodiments, the controller 700 can be incorporated in other housings or components of a pressure mitigation system, including being remotely coupled to a pressure-mitigation device.. Embodiments of the controller 700 can include any subset of the components shown in Figure 7, as well as additional components not illustrated here. For example, some embodiments of the controller 700 include a physical data interface through which data can be transmitted to another computing device. Examples of physical data interfaces include Ethernet ports, Universal Serial Bus (USB) ports, and proprietary ports.

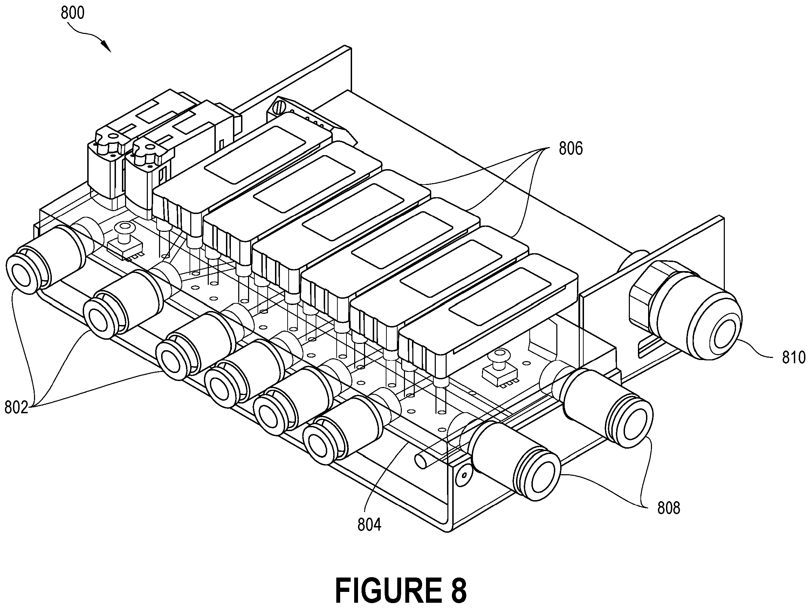

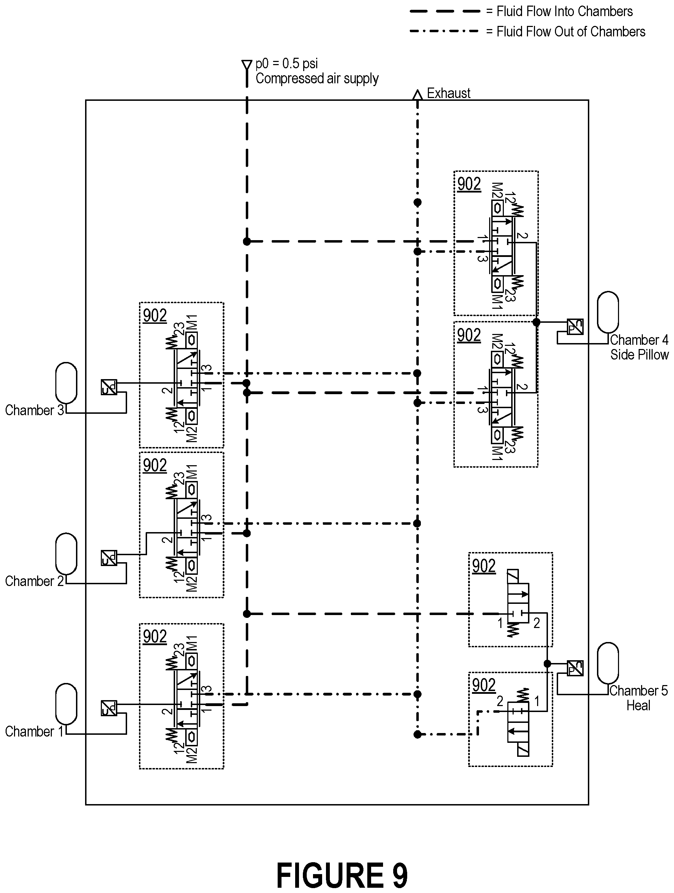

The controller 700 may be connected to a pressure-mitigation apparatus that includes a series of chambers whose pressure can be individually varied. When the pressure-mitigation apparatus is placed between a human body and a support surface, the controller 700 can cause the pressure on an anatomical region of the human body to be varied by controllably inflating one or more chambers, deflating one or more chambers, or any combination thereof. Such action can be accomplished by the manifold 708, which controls fluid flow to the series of chambers of the pressure-mitigation apparatus. The manifold 708 is further described with respect to Figures 8 and 9.

As further described below, transducers mounted in the manifold 708 can generate an electrical signal based on the pressure detected in the chambers of the pressure-mitigation apparatus. Generally, each chamber is associated with a different fluid channel and a different transducer. Accordingly, if the manifold 708 is designed to facilitate fluid flow to a four-chamber pressure-mitigation apparatus, the manifold 708 may include four fluid channels and four transducers. In some embodiments, the manifold 708 may include fewer than four fluid channels and/or transducers or greater than four fluid channels and/or transducers. Pressure data representative of the values of the electrical signals generated by the transducers can be stored, at least temporarily, in the memory 710. In some embodiments, the processor(s) 702 processes the pressure data prior to examination by the analysis module 706. For example, the processor(s) 702 may apply algorithms designed for temporal aligning, artifact removal, and the like.

By examining the pressure data in conjunction with flow data representative of fluid flowing into the controller 700 from the pump(s), the analysis module 706 can control how the chambers of the pressure-mitigation apparatus are inflated and/or deflated. For example, the analysis module 706 may be responsible for separately controlling the set point for fluid flow to each chamber.

Moreover, by examining the pressure data, the analysis module 706 may be able to sense movements of the human body under which the pressure-mitigation apparatus is positioned. These movements may be caused by the patient, another individual (e.g., a caregiver or an operator of the controller 700), or the underlying support surface. The analysis module 706 may apply algorithm(s) to the data representative of these movements (also referred to as "movement data" or "motion data") to identify repetitive movements and/or random movements to better understand the health state of the patient. For example, the analysis module 706 may be able to establish respiration rate or heart rate based on the movements of a patient. Generally, the movement data can be derived from the pressure data. Consequently, the pressure-mitigation apparatus may not actually include any sensors for measuring movement, such as accelerometers, tilt sensors, or gyroscopes.

Following examination of the pressure data, the analysis module 706 may respond in several ways. For example, the analysis module 706 may generate a notification (e.g., an alert) to be transmitted to another computing device by the communication module 704. The other computing device may be associated with a healthcare professional (e.g., a physician or a nurse), a family member of the patient, or some other entity (e.g., a researcher or an insurer). The communication module 704 may communicate with the other computing device via a bi-directional communication protocol, such as Near Field Communication (NFC), wireless USB, Bluetooth, Wi-Fi, a cellular data protocol (e.g., LTE, 3G, 4G, or 5G), or a proprietary point-to-point protocol. As another example, the analysis module 706 may cause the pressure data (or analyses of such data) to be integrated with the electronic health record of the patient. Generally, the electronic health record is maintained in a storage medium accessible to the communication module 704 across a network.

The controller 700 may include a power component 712 able to provide to the other components residing within the housing, as necessary. Examples of power components include rechargeable lithium-ion (Li-Ion) batteries, rechargeable nickel-metal hydride (NiMH) batteries, rechargeable nickel-cadmium (NiCad) batteries, etc. In some embodiments, the controller 700 does not include a power component, and thus must receive power from an external source. In such embodiments, a cable designed to facilitate the transmission of power (e.g., via a physical connection of electrical contacts) may be connected between the power interface 714 of the controller 700 and the external source. The external source may be, for example, an alternating current (AC) power socket or another electronic device.

Figure 8 is an isometric view of a manifold 800 for controlling fluid flow (e.g., air flow) to the chambers of a pressure-mitigation apparatus in accordance with embodiments of the present technology. As described above, a controller can be configured to inflate and/or deflate the chambers of a pressure-mitigation apparatus. To accomplish this, the manifold 800 can guide fluid to the chambers through a series of valves 802. In some embodiments, each valve 802 corresponds to a separate chamber of the pressure-mitigation apparatus. In some embodiments, at least one valve 802 corresponds to multiple chambers of the pressure-mitigation apparatus. In some embodiments, at least one valve 802 is not used during operation. For example, if the pressure-mitigation apparatus includes four chambers, multi-channel tubing may be connected between the pressure-mitigation apparatus and four valves 802 of the manifold 800. In such embodiments, the other valves may remain sealed during operation.

Generally, the valves 802 are piezoelectric valves designed to switch from one state (e.g., an open state) to another state (e.g., a closed state) upon in response to an application of voltage. Piezoelectric valves provide several benefits over other valves, such as linear valves and solenoid-based valves. First, piezoelectric valves do not require holding current to maintain a state. As such, piezoelectric valves generate almost no heat. Second, piezoelectric valves create almost no noise when switching between states, which can be particularly useful in medical settings. Third, piezoelectric valves can be opened and closed in a controlled manner that allows the manifold 800 to precisely approach a given flow rate without overshoot or undershoot. In contrast, the other valves described above must be in either an open state, in which the valve is completely open, or a closed state, in which the valve is completely closed. Fourth, piezoelectric valves require very little power to operate, so a power component of the controller (e.g., power component 712 of Figure 7) may only need to provide 3-6 watts to the manifold 800 at any given time. While embodiments of the manifold 800 may be described in the context of piezoelectric valves, other types of valves, such as linear valves or solenoid-based valves, could be used instead of, or in addition to, piezoelectric valves.

Each piezoelectric valve includes at least one piezoelectric element that acts as an electromechanical transducer. When a voltage is applied to the piezoelectric element, the piezoelectric element is deformed, thereby resulting in mechanical motion (e.g., the opening or closing of a valve). Examples of piezoelectric elements include disc transducers, bender actuators, and piezoelectric stacks.

In some embodiments, the manifold 800 includes one or more transducers 806 and a circuit board 804 that includes one or more integrated circuits (also referred to as "chips") for managing communication with the valves 802 and the transducer(s) 806. Because these local chip(s) reside within the manifold 800 itself, the valves 802 can be digitally controlled in a precise manner. The local chip(s) may also be connected to other components of the controller. For example, the local chip(s) may be connected to processor(s) (e.g., processor(s) 702 of Figure 7) housed within the controller. The transducer(s) 806, meanwhile, can generate an electrical signal based on the pressure of each chamber of the pressure-mitigation apparatus. Generally, each chamber is associated with a different valve 802 and a different transducer 806. Here, for example, the manifold includes six valves 802 capable of interfacing with the pressure-mitigation apparatus, and each of these valves is associated with a corresponding transducer 802. Pressure data representative of the values of the electrical signals generated by the transducer(s) 806 can be provided to other components of the controller for further analysis.

The manifold 800 may also include one or more compressors. In some embodiments each valve 802 of the manifold 800 is fluidically coupled to the same compressor, while in other embodiments each valve 802 of the manifold 800 is fluidically coupled to a different compressor. Each compressor can increase the pressure of fluid (e.g., air) by reducing its volume before guiding the fluid to the pressure-mitigation apparatus.

Fluid produced by a pump may initially be received by the manifold 800 through one or more ingress fluid interfaces 808. As noted above, in some embodiments, a compressor may then increase pressure of the fluid by reducing its volume. Thereafter, the manifold 800 can controllably guide the fluid into the chambers of a pressure-mitigation apparatus through the valves 802. The flow of fluid into each chamber can be controlled by local chip(s) disposed on the circuit board 804. For example, the local chip(s) can dynamically vary the flow of fluid into each chamber in real time by controllably applying voltages to open/close the valves 802.

In some embodiments, the manifold includes one or more egress fluid interfaces 810. The egress fluid interface(s) 810 may be designed for high pressure and high flow to permit rapid deflation of the pressure-mitigation apparatus. For example, upon determining that an operator has provided input indicative of a request to deflate the pressure-mitigation apparatus (or a portion thereof), the manifold 800 may allow fluid to travel back though the valve(s) 802 from the pressure-mitigation apparatus and then out through the egress fluid interface(s) 810. Thus, the egress fluid interface(s) 810 may also be referred to as "exhausts" or "outlets." To provide the input, the operator may interact with a mechanical input component (e.g., mechanical input component 606 of Figure 6A) or a digital input component (e.g., visible on display 610 of Figure 6A).

Figure 9 is a generalized electrical diagram illustrating how the piezoelectric valves 902 of a manifold can separately control fluid flow along multiple channels in accordance with embodiments of the present technology. In Figure 9, the manifold includes seven piezoelectric valves 902. In other embodiments, the manifold may include less than seven valves or more than seven valves. Fluid can be guided by the manifold through the piezoelectric valves 902 to the chambers of a pressure-mitigation apparatus. In Figure 9, the manifold 900 is fluidically connected to a pressure-mitigation apparatus that includes five chambers. However, in other embodiments, the manifold 900 may be fluidically connected to a pressure-mitigation apparatus that includes less than five chambers or more than five chambers.

All of the piezoelectric valves 902 included in the manifold need not necessarily be identical to one another. Piezoelectric valves may be designed for high pressure and low flow, high pressure and high flow, low pressure and low flow, or low pressure and high flow. In some embodiments all of the piezoelectric valves included in the manifold are the same type, while in other embodiments the manifold includes multiple types of piezoelectric valves. For example, piezoelectric valve(s) corresponding to side supports of the pressure-mitigation apparatus may be designed for high pressure and high flow (e.g., to allow for a quick discharge of fluid), but piezoelectric valve(s) corresponding to chambers of the pressure-mitigation apparatus may be designed for high pressure and low flow. Moreover, some piezoelectric valves may support bidirectional fluid flow, while other piezoelectric valves may support unidirectional fluid flow. Generally, if the manifold 900 includes unidirectional piezoelectric valves, each chamber in the pressure-mitigation apparatus is associated with a pair of unidirectional piezoelectric valves to allow fluid flow in either direction. Here, for example, Chambers 1-3 are associated with a single bidirectional piezoelectric valve, Chamber 4 is associated with two bidirectional piezoelectric valves, and Chamber 5 is associated with two unidirectional piezoelectric valves. Further, the manifold of the controller can be configured to inflate and/or deflate each chamber of a pressure-mitigation apparatus to achieve a specified pressure value.

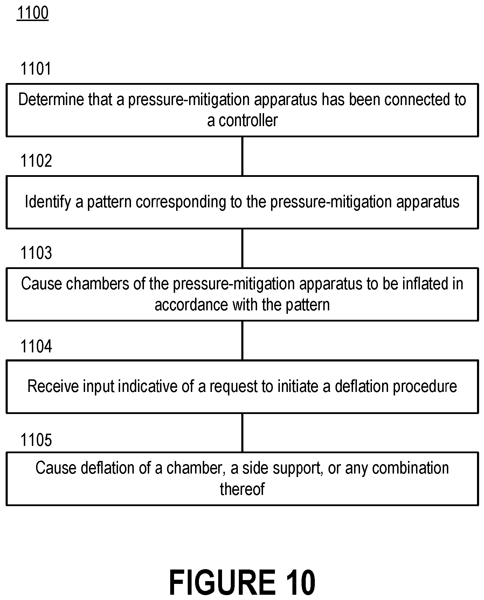

Figure 10 is a flow diagram of a process 1100 for varying pressure in chambers of a pressure-mitigation apparatus positioned between a human body and a support surface in accordance with embodiments of the present technology. By varying the pressure in the chambers, a controller can move the main point of pressure applied by the support surface across the human body. For example, the main point of pressure applied by the support surface to the human body may be moved amongst a plurality of predetermined locations by sequentially varying the pressure in different predetermined subsets of inflatable chambers.

Initially, a controller determines that a pressure-mitigation apparatus has been connected to the controller (step 1101). By monitoring the connection between a fluid interface (e.g., fluid interface 608 of Figure 6B) and the pressure-mitigation apparatus, the controller detects which type of pressure-mitigation apparatus has been connected. In some embodiments, each type of pressure-mitigation apparatus may include a different type of connector. For example, the pressure-mitigation apparatus designed for elongated support surfaces (e.g., pressure-mitigation apparatus 100 of Figures 1A-B) may include a first arrangement of magnets in its connector, while the pressure-mitigation apparatus designed for non-elongated support surfaces (e.g., pressure-mitigation apparatus of Figures 2A-B) may include a second arrangement of magnets in its connector. The controller may determine which type of pressure-mitigation apparatus has been connected based on which magnets have been detected within a specified proximity. As another example, the pressure-mitigation apparatus designed for elongated support surfaces may include a beacon capable of emitting a first electronic signature, while the pressure-mitigation apparatus designed for non-elongated support surfaces may include a beacon capable of emitting a second electronic signature. Examples of beacons include Bluetooth beacons, USB beacons, and infrared beacons. A beacon may be configured to communicate with the controller via a wired communication channel or a wireless communication channel.

The controller identifies a pattern corresponding to the pressure-mitigation apparatus (step 1102). For example, the controller may examine a library of patterns corresponding to different pressure-mitigation apparatuses to identify the appropriate pattern. The library of patterns may be stored in a local memory (e.g., memory 710 of Figure 7) or a remote memory accessible to the controller across a network. As another example, the controller may modify an existing pattern based on the pressure-mitigation apparatus. For instance, the controller may alter the existing pattern responsive to determining that the pattern includes instructions for more chambers than the pressure-mitigation apparatus includes. The controller can then cause the chambers of the pressure-mitigation apparatus to be inflated in accordance with the pattern (step 1103). More specifically, the controller can cause the pressure on one or more anatomical regions of the human body to be varied by controllably inflating one or more chambers, deflating one or more chambers, or any combination thereof.

The controller may receive input indicative of a request to initiate a deflation procedure (step 1104). In some embodiments, the input is associated with an instruction that is manually provided by an operator (e.g., as a result of an interaction with a mechanical input component or a digital input component). For example, the operator may request that the deflation procedure be initiated before the patient is transferred to/from the pressure-mitigation apparatus. As another example, the operator may request that the deflation procedure be initiated before a medical procedure (e.g., cardiopulmonary resuscitation or defibrillation) involving the patient is performed. In other embodiments, the input is associated with an instruction that is automatically generated by the controller. The controller may automatically generate the instruction in response to a specified criterion being satisfied. For example, the controller may automatically generate the instruction when the pressure in a chamber or a side support of the pressure-mitigation apparatus exceeds an upper threshold.

Thereafter, the controller can cause deflation of a chamber, a side support, or any combination thereof (step 1105). More specifically, the controller may instruct a manifold (e.g., manifold 800 of Figure 8) to stop supplying fluid to at least a portion of the pressure-mitigation apparatus and/or open the valve(s) corresponding to the portion of the pressure-mitigation apparatus to allow fluid to escape the pressure-mitigation apparatus.

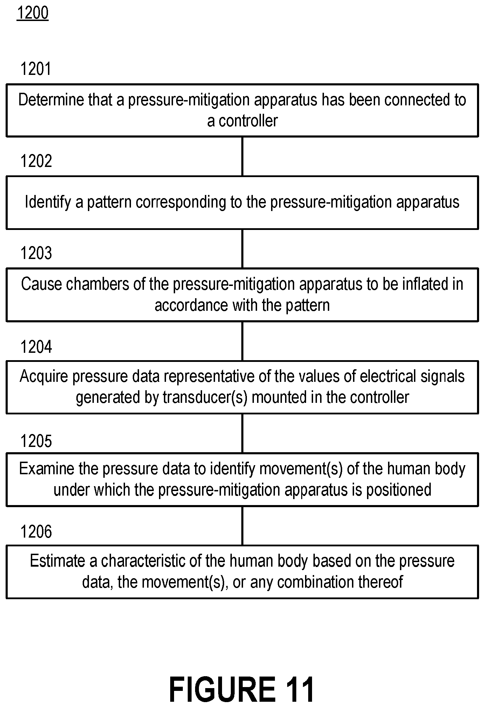

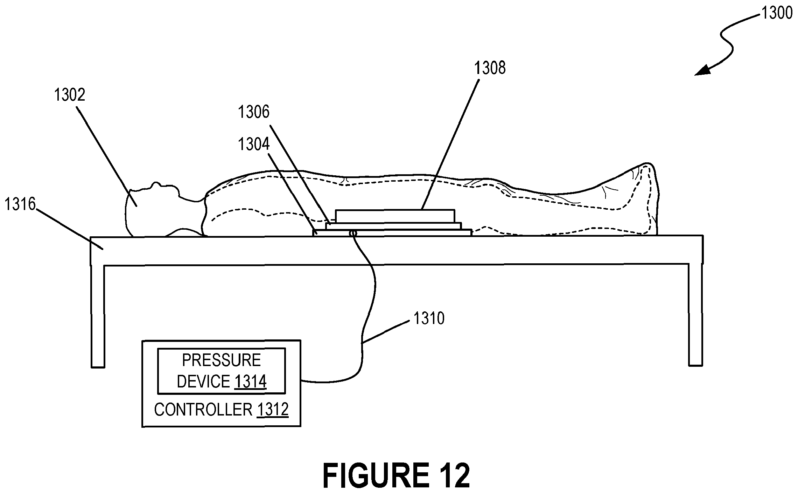

Figure 11 is a flow diagram of a process 1200 for establishing characteristics of the human body supported by a pressure-mitigation apparatus without placing any sensors in direct contact with the human body in accordance with embodiments of the present technology. Steps 1201-1203 of Figure 12 may be at least generally similar to steps 1101 -1103 of Figure 10.

As described above, the controller responsible for managing inflation/deflation of the pressure-mitigation apparatus may include transducer(s) configured to generate an electrical signal based on the pressure of each chamber of the pressure-mitigation apparatus. Accordingly, the controller may acquire pressure data representative of the values of the electrical signals generated by the transducer(s) (step 1204). The controller can then examine the pressure data to identify movement(s) of the human body (step 1205). In some embodiments, the controller can transmit some or all of the pressure data to a remote location (e.g., a central server) for processing or analytics. By constantly monitoring pressures of the chambers of the pressure-mitigation apparatus, the controller can interpret information regarding the movement/location of the human body without requiring the use of sensors in direct contact with the human body.

The monitoring of patient movement via the remote pressure monitoring can be used as an indicator of the patient's mobility status and/or the overall health status of the patient, as well as identify periods of complete immobility, which may indicate a associated with patient movement can also indicate a decline in patient health status or a potential health complication. Remote pressure monitoring can also detect when a patient leaves the bed, chair, or other surface on which the patient-mitigation devices is disposed, and in some embodiments, respond to this movement with an alert or alarm provided locally or to a remote location (e.g., to a caregiver) to draw attention to this movement. This allows patient caregivers to assist when the patient is ambulatory to avoid falls and/or identify falls from the support surface in real time. The remote pressure monitoring data can also be used to determine whether the patient is properly using the device, whether he or she is properly positioned on the pressure-mitigation surface, whether the patient is complying with the prescribed protocol. Based on this information, alerts or alarms transmitted to a remote system accessible by a hospital, caregiver, and/or other individuals involved with the patient's care. The real-time monitoring and analysis of data can provide accurate alarms to alert caregivers, management, and others when the patient is not compliant with the protocol and/or improperly using the device (e.g., positioned incorrectly), thereby promoting appropriate usage and enhancing the benefit of the pressure mitigation system.

The controller (or some other electronic device, such as a mobile phone, laptop computer, or computer server) can estimate a characteristic of the human body based on the pressure data, the movement(s), or any combination thereof (step 1206). For example, the controller may be able to estimate the weight of the human body by examining the pressure data in conjunction with flow data representative of fluid flowing into the controller (e.g., from one or more pumps). As another example, the controller may be able to estimate the respiration rate or heart rate of the human body based on the movements. Accordingly, the controller may be able to understand certain aspects of the health state of the human body, such as mobility state, in a noninvasive manner.

Unless contrary to physical possibility, it is envisioned that the steps described above may be performed in various sequences and combinations. For example, the controller may be configured to perform the process 1100 of Figure 10 and the process 1200 of Figure 11 simultaneously. Other steps may also be included in some embodiments. For example, the controller may cause a notification to be transmitted to another computing device in certain situations (e.g., upon discovering movement indicative of discomfort in a specified anatomical region of the human body, movement indicative of an attempt to leave the pressure-mitigation apparatus, or a complete lack of movement for a specified period of time).

### Selected Embodiments of Pressure-Mitigation Systems

Figure 12 is a partially schematic side view of a pressure-mitigation system 1300 (or simply "system") for orienting an individual 1302 over a pressure-mitigation apparatus 1306 in accordance with embodiments of the present technology. The system 1300 can include a pressure-mitigation apparatus 1306 that include side supports 1308, an attachment apparatus 1304, a pressure device 1314, and a controller 1312. As shown in Figure 12, the attachment apparatus 1304 may be responsible for securing the pressure-m itigation apparatus 1306 to the support surface 1316. Further examples of the attachment apparatus are discussed in detail with respect to Figures 1-3, and further examples of the pressure-mitigation apparatus are discussed in detail with respect to Figures 4A-6.

In this embodiment, the pressure-mitigation apparatus 1306 includes a pair of optional elevated side supports 1308 that extend longitudinally along opposing sides of the pressure-mitigation apparatus 1306. The pressure-mitigation apparatus 1306 includes a series of chambers interconnected on a base material. As further described above, the chambers may be arranged in a geometric pattern designed to mitigate the pressure applied to a specific anatomical region by the support surface 1316.

The elevated side supports 1308 can be configured to actively orient the specific anatomical region of the individual 1302 over the series of chambers. For example, the elevated side supports 1308 may be responsible for actively orienting the specific anatomical region widthwise over the epicenter of the geometric pattern. As shown in Figure 10, the specific anatomical region may be the sacral region. However, the specific anatomical region could be any region of the body that is susceptible to pressure, and thus the formation of pressure ulcers. The elevated side supports 1308 may be configured to be ergonomically comfortable. For example, the elevated side supports 1308 may include a recess designed to accommodate the forearm, which permits pressure to be offloaded from the elbow.

The elevated side supports 1308 may be significantly larger in size as compared to the chambers of the pressure-mitigation apparatus 1306. Accordingly, the elevated side supports 1308 may create a barrier that restricts lateral movement of the individual 1302. In some embodiments, the elevated side supports are approximately 2-3 inches taller in height as compared to the average height of an inflated chamber. Because the elevated side supports 1306 straddle the individual 1302, the elevated side supports 1308 can act as barriers for maintaining the position of the individual 1302 on top of the pressure-mitigation apparatus 1306. In some embodiments, the elevated side supports 1308 may be omitted.

In some embodiments, the inner side walls of the elevated side supports 1308 form, following inflation, a firm surface at a steep angle of orientation with respect to the pressure-mitigation apparatus 1306. For example, the inner side walls may be on a plane of approximately 115 degrees, plus or minus 24 degrees, from the plane of the pressure-mitigation apparatus 1306. These steep inner side walls can form a channel that naturally positions the individual 1302 over the chambers of the pressure-mitigation apparatus 1306. Thus, inflation of the elevated side supports 1308 may actively force the individual 1302 into the appropriate position for mitigating pressure by orienting the individual 1302 in the correct location with respect to the chambers of the pressure-mitigation apparatus 1306.

After the initial inflation cycle has been completed, the pressure of each elevated side support 1308 may be lessened to increase comfort and prevent excessive force against the lateral sides of the individual 1302. Oftentimes a medical professional (e.g., a physician, nurse, or caregiver) will be present during the initial inflation cycle to ensure the elevated side supports 1308 properly position the individual 1302 over the pressure-mitigation apparatus 1306.

The controller 1312 can be configured to regulate the pressure of each chamber included in the pressure-mitigation apparatus 1306 and/or each elevated side support 1308 via a pressure device 1314 (e.g., an air pump) and multi-channel tubing 1310. For example, the chambers may be controlled in a specific pattern to preserve blood flow and reduce pressure applied to the individual 1302 when inflated (pressurized) and deflated (depressurized) in a coordinated fashion by the controller 1312. The multi-channel tubing 1310 may be connected between the pressure-mitigation apparatus 1306 and the pressure device 1314. Accordingly, the pressure-mitigation apparatus 1306 may be fluidically coupled to a first end of the multi-channel tubing 1310, and the pressure device 1314 may be fluidically coupled to a second end of the multi-channel tubing 1310.

### Processing System

Figure 13 is a block diagram illustrating a processing system 1400 in which at least some operations described herein can be implemented. For example, some components of the processing system 1400 may be hosted on a controller (e.g., controller 1312 of Figure 12) responsible for controlling a pressure-mitigation apparatus (e.g., pressure-mitigation apparatus 1306 of Figure 12).

The processing system 1400 may include one or more central processing units ("processors") 1402, main memory 1406, non-volatile memory 1410, network adapter 1412 (e.g., network interface), video display 1418, input/output devices 1420, control device 1422 (e.g., keyboard and pointing devices), drive unit 1424 including a storage medium 1426, and signal generation device 1430 that are communicatively connected to a bus 1416. The bus 1416 is illustrated as an abstraction that represents one or more physical buses and/or point-to-point connections that are connected by appropriate bridges, adapters, or controllers. The bus 1416, therefore, can include a system bus, a Peripheral Component Interconnect (PCI) bus or PCI-Express bus, a HyperTransport or industry standard architecture (ISA) bus, a small computer system interface (SCSI) bus, a universal serial bus (USB), IIC (I2C) bus, or an Institute of Electrical and Electronics Engineers (IEEE) standard 1394 bus (also referred to as "Firewire").

The processing system 1400 may share a similar computer processor architecture as that of a desktop computer, tablet computer, personal digital assistant (PDA), mobile phone, game console, music player, wearable electronic device (e.g., a watch or fitness tracker), network-connected ("smart") device (e.g., a television or home assistant device), virtual/augmented reality systems (e.g., a head-mounted display), or another electronic device capable of executing a set of instructions (sequential or otherwise) that specify action(s) to be taken by the processing system 1400.

While the main memory 1406, non-volatile memory 1410, and storage medium 1426 (also called a "machine-readable medium") are shown to be a single medium, the term "machine-readable medium" and "storage medium" should be taken to include a single medium or multiple media (e.g., a centralized/distributed database and/or associated caches and servers) that store one or more sets of instructions 1428. The term "machine-readable medium" and "storage medium" shall also be taken to include any medium that is capable of storing, encoding, or carrying a set of instructions for execution by the processing system 1400.

In general, the routines executed to implement the embodiments of the disclosure may be implemented as part of an operating system or a specific application, component, program, object, module, or sequence of instructions (collectively referred to as "computer programs"). The computer programs typically comprise one or more instructions (e.g., instructions 1404, 1408, 1428) set at various times in various memory and storage devices in a computing device. When read and executed by the one or more processors 1402, the instruction(s) cause the processing system 1400 to perform operations to execute elements involving the various aspects of the disclosure.

Moreover, while embodiments have been described in the context of fully functioning computing devices, those skilled in the art will appreciate that the various embodiments are capable of being distributed as a program product in a variety of forms. The disclosure applies regardless of the particular type of machine or computer-readable media used to actually effect the distribution.

Further examples of machine-readable storage media, machine-readable media, or computer-readable media include recordable-type media such as volatile and non-volatile memory devices 1410, floppy and other removable disks, hard disk drives, optical disks (e.g., Compact Disk Read-Only Memory (CD-ROMS), Digital Versatile Disks (DVDs)), and transmission-type media such as digital and analog communication links.

The network adapter 1412 enables the processing system 1400 to mediate data in a network 1414 with an entity that is external to the processing system 1400 through any communication protocol supported by the processing system 1400 and the external entity. The network adapter 1412 can include a network adaptor card, a wireless network interface card, a router, an access point, a wireless router, a switch, a multilayer switch, a protocol converter, a gateway, a bridge, bridge router, a hub, a digital media receiver, and/or a repeater.

The network adapter 1412 may include a firewall that governs and/or manages permission to access/proxy data in a computer network, and tracks varying levels of trust between different machines and/or applications. The firewall can be any number of modules having any combination of hardware and/or software components able to enforce a predetermined set of access rights between a particular set of machines and applications, machines and machines, and/or applications and applications (e.g., to regulate the flow of traffic and resource sharing between these entities). The firewall may additionally manage and/or have access to an access control list that details permissions including the access and operation rights of an object by an individual, a machine, and/or an application, and the circumstances under which the permission rights stand.

The techniques introduced here can be implemented by programmable circuitry (e.g., one or more microprocessors), software and/or firmware, special-purpose hardwired (i.e., non-programmable) circuitry, or a combination of such forms. Special-purpose circuitry can be in the form of one or more application-specific integrated circuits (ASICs), programmable logic devices (PLDs), field-programmable gate arrays (FPGAs), etc.

### Conclusion

The above detailed descriptions of embodiments of the technology are not intended to be exhaustive or to limit the technology to the precise form disclosed above. Although specific embodiments of, and examples for, the technology are described above for illustrative purposes, various equivalent modifications are possible within the scope of the technology as those skilled in the relevant art will recognize. For example, although steps are presented in a given order, alternative embodiments may perform steps in a different order. The various embodiments described herein may also be combined to provide further embodiments.

From the foregoing, it will be appreciated that specific embodiments of the technology have been described herein for purposes of illustration, but well-known structures and functions have not been shown or described in detail to avoid unnecessarily obscuring the description of the embodiments of the technology. Where the context permits, singular or plural terms may also include the plural or singular term, respectively.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein, if they fall under the scope defined by the appended claims.

## Claims

1. A controller comprising:
a structural body (602), wherein the structural body (602) comprises:
an ingress or first fluid interface configured to be fluidically coupled to a pump, and
an egress or second fluid interface (608) configured to be fluidically coupled to a pressure-mitigation pad (100, 200, 300, 400, 502a, 502b, 1306) that includes a plurality of chambers (508) that are selectively inflatable and is disposed between a human body (504) and a surface (500);
one or more sensors that are arranged proximate to the egress interface;
a processor (702); and
a memory (710) that includes instructions for regulating a flow of air provided by
the pump to inflate the plurality of chambers (508) of the pressure-mitigation pad (502a) in a controlled manner,
wherein the instructions, when executed by the processor (702), cause the processor (702) to:
determine, based on analysis of outputs produced by the one or more sensors, that the pressure-mitigation pad (100, 200, 300, 400, 502a, 502b, 1306) is connected to the egress interface (608),
identify a programmable pattern corresponding to the pressure-mitigation pad (100, 200, 300, 400, 502a, 502b, 1306), and
cause the plurality of chambers (508) of the pressure-mitigation pad (100, 200, 300, 400, 502a, 502b, 1306) to be inflated to varying degrees in accordance with the programmable pattern, thereby shifting a point of contact pressure applied by the surface (500) to the human body (504) over time.

2. The controller of claim 1, further comprising:
a manifold (708, 800), wherein the manifold (708, 800) comprises:
a plurality of valves (802),
wherein each valve (802) is configured to regulate airflow into a corresponding chamber of the plurality of chambers (508) of the pressure-mitigation pad (100, 200, 300, 400, 502a, 502b, 1306); and
a plurality of transducers (806),
wherein each transducer (806) is configured to monitor pressure of a corresponding chamber of the plurality of chambers (508) of the pressure-mitigation pad (100, 200, 300, 400, 502a, 502b, 1306).

3. The controller of claim 1, wherein the egress interface (810) includes a plurality of channels, and wherein each channel of the plurality of channels corresponds to a different chamber of the plurality of chambers (508) of the pressure-mitigation pad (100, 200, 300, 400, 502a, 502b, 1306).

4. The controller of claim 1, further comprising:
a mechanical input component (606) operatively coupled to the processor (702), wherein upon receiving input indicative of an interaction with the mechanical input component (606), the processor (702) is configured to identify an appropriate instruction for regulating the flow of air; and
a display (610) for presenting information related to the flow of air, the pressure-mitigation pad (100, 200, 300, 400, 502a, 502b, 1306), an individual presently undergoing treatment, or any combination thereof.

5. The controller of claim 1, wherein, upon deployment of the pressure-mitigation pad (100, 200, 300, 400, 502a, 502b, 1306), the processor (702) causes the plurality of chambers (508) to be in a naturally inflated state,
wherein the processor (702) is configured to mitigate contact pressure on an anatomical region of the human body (504) in accordance with the programmable pattern, and
wherein the programmable pattern causes contact pressure on the anatomical region to be lessened by prompting the processor (702) to cause deflation of at least one chamber positioned beneath the anatomical region.

6. The controller of claim 1, wherein, upon deployment of the pressure-mitigation pad (100, 200, 300, 400, 502a, 502b, 1306), the processor (702) causes the plurality of chambers (508) to be in a naturally deflated state,
wherein the processor (702) is configured to mitigate contact pressure on an anatomical region of the human body (504) in accordance with the programmable pattern, and
wherein the programmable pattern causes contact pressure on the anatomical region to be lessened by prompting the processor (702) to cause inflation of at least one chamber positioned adjacent to the anatomical region.

7. The controller of claim 2, wherein the manifold further comprises:
a further egress interface (810) through which air can be discharged into an ambient environment; and
a compressor configured to pressurize air received from the pump before delivery to the plurality of chambers (508) of the pressure-mitigation pad(100, 200, 300, 400, 502a, 502b, 1306) via the plurality of valves (902).

8. The controller of claim 2, wherein the plurality of valves comprises bidirectional valves that permit airflow in multiple directions, unidirectional valves that only permit airflow in a single direction, or a combination thereof.

9. A method comprising:
determining, by a controller according to any of the previous claims, that a pressure-mitigation pad (100, 200, 300, 400, 502a, 502b, 1306) disposed between a human body (504) and a surface (500) has been fluidically coupled to an egress or second fluid interface (608) of the controller based on analysis of outputs produced by one or more sensors arranged proximate to the egress interface,
wherein the pressure-mitigation pad (100, 200, 300, 400, 502a, 502b, 1306) includes a plurality of chambers (508);
identifying, by the controller, a programmable pattern corresponding to the pressure-mitigation pad (100, 200, 300, 400, 502a, 502b, 1306); and
causing, by the controller, the plurality of chambers (508) of the pressure-mitigation pad (100, 200, 300, 400, 502a, 502b, 1306) to be inflated to varying degrees in accordance with the programmable pattern, thereby shifting a point of contact pressure applied by the surface (500) to the human body (504) over time.

10. The method of claim 9, wherein said determining step comprises:
detecting, by the controller, how many magnets, if any, are located within a specified proximity of the egress interface based on the outputs produced by the one or more sensors;
wherein the method further comprises:
identifying, by the controller, a type of pressure-mitigation apparatus pad (100, 200, 300, 400, 502a, 502b, 1306) fluidically coupled to the egress interface based on a count of the magnets located within the specified proximity; and
wherein the programmable pattern corresponds to the type of pressure-mitigation pad(100, 200, 300, 400, 502a, 502b, 1306).

11. The method of claim 9, further comprising:
receiving, by the controller, input indicative of a request to initiate a deflation procedure; and
causing, by the controller, deflation of fluid from at least one chamber of the pressure-mitigation pad (100, 200, 300, 400, 502a, 502b, 1306) through a further egress interface into an ambient environment;
wherein the request is:
manually provided by an operator through an interaction with a mechanical input component of the controller, or
automatically provided by the controller responsive to a determination that the pressure of a chamber of the pressure-mitigation pad (502a) exceeds an upper threshold.

12. The method of claim 9, further comprising:
acquiring, by the controller, data indicative of the pressure of each chamber of the plurality of chambers (508) of the pressure-mitigation pad (100, 200, 300, 400, 502a, 502b, 1306);
examining, by the controller, the data to identify movements, if any, performed by the human body (504);
determining, by the controller, that the human body (504) has performed no movements for a specified period of time; and
causing, by the controller, a notification to be transmitted to an electronic device, wherein the notification specifies that no movements have been performed by the human body (504) for the specified period of time.

13. The method of claim 9 further comprising:
acquiring, by the controller, data indicative of the pressure of each chamber of the plurality of chambers (508) of the pressure-mitigation pad (502a);
examining, by the controller, the data to identify movements, if any, performed by the human body (504);
determining, by the controller, that the movements performed by the human body (504) match a specified pattern; and
causing, by the controller, a notification to be transmitted to an electronic device, wherein the notification specifies that the movements performed by the human body (504) match the specified pattern.

14. The method of claim 9, further comprising:
acquiring, by the controller, data indicative of the pressure of each chamber of the plurality of chambers (508) of the pressure-mitigation apparatus (100, 200, 300, 400, 502a, 502b, 1306); and
estimating, by the controller, a characteristic of the human body (504) based on the data,
wherein the characteristic is a weight, a heart rate, or a respiration rate.

## Patentansprüche

1. Steuerung, umfassend:
einen Strukturkörper (602), wobei der Strukturkörper (602) umfasst:
eine Eingangs- oder erste Fluidschnittstelle, die konfiguriert ist, um mit einer Pumpe fluidisch gekoppelt zu werden, und
eine Ausgangs- oder zweite Fluidschnittstelle (608), die konfiguriert ist, um mit einem Druckminderungskissen (100, 200, 300, 400, 502a, 502b, 1306) fluidisch gekoppelt zu werden, das eine Vielzahl von Kammern (508), die selektiv aufblasbar sind, einschließt und zwischen einem menschlichen Körper (504) und einer Oberfläche (500) eingerichtet ist;
einen oder mehrere Sensoren, die nahe der Ausgangsschnittstelle aufgestellt sind;
einen Prozessor (702); und
einen Speicher (710), der Anweisungen zum Regulieren eines Stroms von Luft, der durch die Pumpe bereitgestellt wird, einschließt, um die Vielzahl von Kammern (508) des Druckminderungskissens (502a) auf eine gesteuerte Weise aufzublasen,
wobei die Anweisungen, wenn sie durch den Prozessor (702) durchgeführt werden, den Prozessor (702) veranlassen zum:
Bestimmen, basierend auf einer Analyse von Ausgaben, die durch den einen oder die mehreren Sensoren erzeugt werden, dass das Druckminderungskissen (100, 200, 300, 400, 502a, 502b, 1306) mit der Ausgangsschnittstelle (608) verbunden ist,
Identifizieren eines programmierbaren Musters, das dem Druckminderungskissen (100, 200, 300, 400, 502a, 502b, 1306) entspricht, und
Veranlassen, dass die Vielzahl von Kammern (508) des Druckminderungskissens (100, 200, 300, 400, 502a, 502b, 1306) gemäß dem programmierbaren Muster in unterschiedlichen Ausmaßen aufgeblasen werden, wobei dadurch ein Kontaktdruckpunkt, der durch die Oberfläche (500) auf den menschlichen Körper (504) ausgeübt wird, im Laufe der Zeit verschoben wird.

2. Steuerung nach Anspruch 1, ferner umfassend:
einen Verteiler (708, 800), wobei der Verteiler (708, 800) umfasst:
eine Vielzahl von Ventilen (802),
wobei jedes Ventil (802) konfiguriert ist, um einen Luftstrom in eine entsprechende Kammer der Vielzahl von Kammern (508) des Druckminderungskissens (100, 200, 300, 400, 502a, 502b, 1306) zu regulieren; und
eine Vielzahl von Aufnehmern (806),
wobei jeder Aufnehmer (806) konfiguriert ist, um einen Druck einer entsprechenden Kammer der Vielzahl von Kammern (508) des Druckminderungskissens (100, 200, 300, 400, 502a, 502b, 1306) zu überwachen.

3. Steuerung nach Anspruch 1, wobei die Ausgangsschnittstelle (810) eine Vielzahl von Kanälen einschließt und wobei jeder Kanal der Vielzahl von Kanälen einer anderen Kammer der Vielzahl von Kammern (508) des Druckminderungskissens (100, 200, 300, 400, 502a, 502b, 1306) entspricht.

4. Steuerung nach Anspruch 1, ferner umfassend:
eine mechanische Eingabekomponente (606), die mit dem Prozessor (702) wirkverbunden ist,
wobei bei einem Erhalten einer Eingabe, die eine Interaktion mit der mechanischen Eingabekomponente (606) angibt, der Prozessor (702) konfiguriert ist, um eine geeignete Anweisung zum Regulieren des Stroms von Luft zu identifizieren; und
eine Anzeige (610) zum Darstellen von Informationen, sie sich auf den Strom von Luft, das Druckminderungskissen (100, 200, 300, 400, 502a, 502b, 1306), eine Person, die sich derzeit einer Behandlung unterzieht, oder eine beliebige Kombination davon beziehen.

5. Steuerung nach Anspruch 1, wobei, bei einer Entfaltung des Druckminderungskissens (100, 200, 300, 400, 502a, 502b, 1306), der Prozessor (702) veranlasst, dass sich die Vielzahl von Kammern (508) in einem natürlich aufgeblasenen Zustand befinden,
wobei der Prozessor (702) konfiguriert ist, um einen Kontaktdruck auf eine anatomische Region des menschlichen Körpers (504) gemäß dem programmierbaren Muster zu mindern, und
wobei das programmierbare Muster veranlasst, dass der Kontaktdruck auf die anatomische Region verringert wird durch Auffordern des Prozessors (702), eine Entleerung mindestens einer Kammer, die unterhalb der anatomischen Region positioniert ist, zu veranlassen.

6. Steuerung nach Anspruch 1, wobei, bei der Entfaltung des Druckminderungskissens (100, 200, 300, 400, 502a, 502b, 1306), der Prozessor (702) veranlasst, dass sich die Vielzahl von Kammern (508) in einem natürlich entleerten Zustand befinden,
wobei der Prozessor (702) konfiguriert ist, um den Kontaktdruck auf eine anatomische Region des menschlichen Körpers (504) gemäß dem programmierbaren Muster zu mindern, und
wobei das programmierbare Muster veranlasst, dass der Kontaktdruck auf die anatomische Region verringert wird durch Auffordern des Prozessors (702), ein Aufblasen mindestens einer Kammer, die angrenzend an die anatomische Region positioniert ist, zu veranlassen.

7. Steuerung nach Anspruch 2, wobei der Verteiler ferner umfasst:
eine weitere Ausgangsschnittstelle (810), über die Luft in ein umgebendes Umfeld ausgestoßen werden kann; und
einen Kompressor, der konfiguriert ist, um Luft, die von der Pumpe erhalten wird, vor einer Zufuhr zu der Vielzahl von Kammern (508) des Druckminderungskissens (100, 200, 300, 400, 502a, 502b, 1306) mittels der Vielzahl von Ventilen (902) unter Druck zu setzen.

8. Steuerung nach Anspruch 2, wobei die Vielzahl von Ventilen bidirektionale Ventile, die den Luftstrom in eine Mehrzahl von Richtungen ermöglichen, unidirektionale Ventile, die nur den Luftstrom in eine einzige Richtung ermöglichen, oder eine Kombination davon umfassen.

9. Verfahren, umfassend:
Bestimmen, durch eine Steuerung nach einem der vorstehenden Ansprüche,
dass ein Druckminderungskissen (100, 200, 300, 400, 502a, 502b, 1306), das zwischen einem menschlichen Körper (504) und einer Oberfläche (500) eingerichtet ist, mit einer Ausgangs- oder zweiten Fluidschnittstelle (608) der Steuerung fluidisch gekoppelt wurde, basierend auf der Analyse von Ausgaben, die durch einen oder mehrere Sensoren, die nahe der Ausgangsschnittstelle aufgestellt sind, erzeugt werden,
wobei das Druckminderungskissen (100, 200, 300, 400, 502a, 502b, 1306) eine Vielzahl von Kammern (508) einschließt;
Identifizieren, durch die Steuerung, eines programmierbaren Musters, das dem Druckminderungskissen (100, 200, 300, 400, 502a, 502b, 1306) entspricht; und
Veranlassen, durch die Steuerung, dass die Vielzahl von Kammern (508) des Druckminderungskissens (100, 200, 300, 400, 502a, 502b, 1306) gemäß dem programmierbaren Muster in unterschiedlichen Ausmaßen aufgeblasen werden, wobei dadurch ein Kontaktdruckpunkt, der durch die Oberfläche (500) auf den menschlichen Körper (504) ausgeübt wird, im Laufe der Zeit verschoben wird.

10. Verfahren nach Anspruch 9, wobei der Bestimmungsschritt umfasst:
Detektieren, durch die Steuerung, wie viele Magnete, falls vorhanden, innerhalb einer spezifizierten Nähe der Ausgangsschnittstelle gelegen sind, basierend auf den Ausgaben, die durch den einen oder die mehreren Sensoren erzeugt werden;
wobei das Verfahren ferner umfasst:
Identifizieren, durch die Steuerung, einer Art von Druckminderungseinrichtungskissen (100, 200, 300, 400, 502a, 502b, 1306), das mit der Ausgangsschnittstelle fluidisch gekoppelt ist, basierend auf einer Anzahl der Magnete, die innerhalb der spezifizierten Nähe gelegen sind; und
wobei das programmierbare Muster der Art des Druckminderungskissens (100, 200, 300, 400, 502a, 502b, 1306) entspricht.

11. Verfahren nach Anspruch 9, ferner umfassend:
Erhalten, durch die Steuerung, der Eingabe, die eine Anforderung, einen Entleerungsvorgang zu initiieren, angibt; und
Veranlassen, durch die Steuerung, der Entleerung von Fluid von mindestens einer Kammer des Druckminderungskissens (100, 200, 300, 400, 502a, 502b, 1306) über eine weitere Ausgangsschnittstelle in ein umgebendes Umfeld;
wobei die Anforderung:
durch einen Bediener über eine Interaktion mit einer mechanischen Eingabekomponente der Steuerung manuell bereitgestellt oder
durch die Steuerung als Reaktion auf eine Bestimmung, dass der Druck einer Kammer des Druckminderungskissens (502a) einen oberen Schwellenwert überschreitet, automatisch bereitgestellt wird.

12. Verfahren nach Anspruch 9, ferner umfassend:
Erfassen, durch die Steuerung, von Daten, die den Druck jeder Kammer der Vielzahl von Kammern (508) des Druckminderungskissens (100, 200, 300, 400, 502a, 502b, 1306) angeben;
Untersuchen, durch die Steuerung, der Daten, um Bewegungen, falls vorhanden, die durch den menschlichen Körper (504) ausgeführt werden, zu identifizieren;
Bestimmen, durch die Steuerung, dass der menschliche Körper (504) keine Bewegungen für einen spezifizierten Zeitraum ausgeführt hat; und
Veranlassen, durch die Steuerung, einer Benachrichtigung, die an eine elektronische Vorrichtung zu übertragen ist,
wobei die Benachrichtigung spezifiziert, dass keine Bewegungen durch den menschlichen Körper (504) für den spezifizierten Zeitraum ausgeführt wurden.

13. Verfahren nach Anspruch 9, ferner umfassend:
Erfassen, durch die Steuerung, von Daten, die den Druck jeder Kammer der Vielzahl von Kammern (508) des Druckminderungskissens (502a) angeben;
Untersuchen, durch die Steuerung, der Daten, um Bewegungen, falls vorhanden, die durch den menschlichen Körper (504) ausgeführt werden, zu identifizieren;
Bestimmen, durch die Steuerung, dass die Bewegungen, die durch den menschlichen Körper (504) ausgeführt werden, mit einem spezifizierten Muster übereinstimmen; und
Veranlassen, durch die Steuerung, einer Benachrichtigung, die an eine elektronische Vorrichtung zu übertragen ist,
wobei die Benachrichtigung spezifiziert, dass die Bewegungen, die durch den menschlichen Körper (504) ausgeführt werden, mit dem spezifizierten Muster übereinstimmen.

14. Verfahren nach Anspruch 9, ferner umfassend:
Erfassen, durch die Steuerung, von Daten, die den Druck jeder Kammer der Vielzahl von Kammern (508) der Druckminderungseinrichtung (100, 200, 300, 400, 502a, 502b, 1306) angeben; und
Schätzen, durch die Steuerung, einer Eigenschaft des menschlichen Körpers (504) basierend auf den Daten,
wobei die Eigenschaft ein Gewicht, eine Herzfrequenz oder eine Atemfrequenz ist.

## Revendications

1. Dispositif de commande comprenant :
un corps structurel (602), dans lequel le corps structurel (602) comprend :
une interface d'entrée ou première interface de fluide conçue pour être raccordée fluidiquement à une pompe, et
une interface de sortie ou seconde interface de fluide (608) conçue pour être raccordée fluidiquement à un tampon d'atténuation de pression (100, 200, 300, 400, 502a, 502b, 1306) qui comporte une pluralité de chambres (508) qui sont gonflables sélectivement et est disposé entre un corps humain (504) et une surface (500) ;
un ou plusieurs capteurs qui sont agencés à proximité de l'interface de sortie ;
un processeur (702) ; et
une mémoire (710) qui comporte des instructions permettant de réguler un écoulement d'air fourni par la pompe pour gonfler la pluralité de chambres (508) du tampon d'atténuation de pression (502a) d'une manière contrôlée,
dans lequel les instructions, lorsqu'elles sont exécutées par le processeur (702), amènent le processeur (702) à :
déterminer, sur la base d'une analyse de sorties produites par le ou les capteurs, que le tampon d'atténuation de pression (100, 200, 300, 400, 502a, 502b, 1306) est relié à l'interface de sortie (608),
identifier un modèle programmable correspondant au tampon d'atténuation de pression (100, 200, 300, 400, 502a, 502b, 1306), et
amener la pluralité de chambres (508) du tampon d'atténuation de pression (100, 200, 300, 400, 502a, 502b, 1306) à être gonflées à des degrés variables conformément au modèle programmable, permettant ainsi de décaler un point de pression de contact appliquée par la surface (500) au corps humain (504) au cours du temps.

2. Dispositif de commande selon la revendication 1, comprenant en outre :
un collecteur (708, 800), dans lequel le collecteur (708, 800) comprend :
une pluralité de vannes (802),
dans lequel chaque vanne (802) est conçue pour réguler un écoulement d'air dans une chambre correspondante de la pluralité de chambres (508) du tampon d'atténuation de pression (100, 200, 300, 400, 502a, 502b, 1306) ; et
une pluralité de transducteurs (806),
dans lequel chaque transducteur (806) est configuré pour surveiller la pression d'une chambre correspondante de la pluralité de chambres (508) du tampon d'atténuation de pression (100, 200, 300, 400, 502a, 502b, 1306).

3. Dispositif de commande selon la revendication 1, dans lequel l'interface de sortie (810) comporte une pluralité de canaux, et dans lequel chaque canal de la pluralité de canaux correspond à une chambre différente de la pluralité de chambres (508) du tampon d'atténuation de pression (100, 200, 300, 400, 502a, 502b, 1306).

4. Dispositif de commande selon la revendication 1, comprenant en outre :
un composant d'entrée mécanique (606) couplé fonctionnellement au processeur (702),
dans lequel, lors de la réception d'une entrée indiquant une interaction avec le composant d'entrée mécanique (606), le processeur (702) est configuré pour identifier une instruction appropriée pour réguler l'écoulement d'air ; et
un dispositif d'affichage (610) permettant de présenter des informations relatives à l'écoulement d'air, au tampon d'atténuation de pression (100, 200, 300, 400, 502a, 502b, 1306), à un individu actuellement en cours de traitement, ou à toute combinaison de ceux-ci.

5. Dispositif de commande selon la revendication 1, dans lequel, lors du déploiement du tampon d'atténuation de pression (100, 200, 300, 400, 502a, 502b, 1306), le processeur (702) amène la pluralité de chambres (508) à être dans un état naturellement gonflé,
dans lequel le processeur (702) est configuré pour atténuer la pression de contact sur une région anatomique du corps humain (504) conformément au modèle programmable, et
dans lequel le modèle programmable amène la pression de contact sur la région anatomique à être atténuée en invitant le processeur (702) à provoquer un dégonflage d'au moins une chambre positionnée sous la région anatomique.

6. Dispositif de commande selon la revendication 1, dans lequel, lors du déploiement du tampon d'atténuation de pression (100, 200, 300, 400, 502a, 502b, 1306), le processeur (702) amène la pluralité de chambres (508) à être dans un état naturellement dégonflé,
dans lequel le processeur (702) est configuré pour atténuer la pression de contact sur une région anatomique du corps humain (504) conformément au modèle programmable, et
dans lequel le modèle programmable amène la pression de contact sur la région anatomique à être réduite en invitant le processeur (702) à provoquer le gonflage d'au moins une chambre positionnée adjacente à la région anatomique.

7. Dispositif de commande selon la revendication 2, dans lequel le collecteur comprend en outre :
une interface de sortie (810) supplémentaire à travers laquelle de l'air peut être déchargé dans un environnement ambiant ; et
un compresseur configuré pour mettre sous pression de l'air reçu de la pompe avant distribution à la pluralité de chambres (508) du tampon d'atténuation de pression (100, 200, 300, 400, 502a, 502b, 1306) par l'intermédiaire de la pluralité de vannes (902).

8. Dispositif de commande selon la revendication 2, dans lequel la pluralité de vannes comprend des vannes bidirectionnelles qui permettent un écoulement d'air dans de multiples directions, des vannes unidirectionnelles qui permettent uniquement un écoulement d'air dans une seule direction, ou une combinaison de celles-ci.

9. Procédé comprenant :
le fait de déterminer, par un dispositif de commande selon l'une quelconque des revendications précédentes,
qu'un tampon d'atténuation de pression (100, 200, 300, 400, 502a, 502b, 1306) disposé entre un corps humain (504) et une surface (500) a été raccordé fluidiquement à une interface de sortie ou seconde interface de fluide (608) du dispositif de commande sur la base d'une analyse de sorties produites par un ou plusieurs capteurs disposés à proximité de l'interface de sortie,
dans lequel le tampon d'atténuation de pression (100, 200, 300, 400, 502a, 502b, 1306) comporte une pluralité de chambres (508) ;
l'identification, par le dispositif de commande, d'un modèle programmable correspondant au tampon d'atténuation de pression (100, 200, 300, 400, 502a, 502b, 1306) ; et
le fait d'amener, par le dispositif de commande, la pluralité de chambres (508) du tampon d'atténuation de pression (100, 200, 300, 400, 502a, 502b, 1306) à être gonflées à des degrés variables conformément au modèle programmable, permettant ainsi de décaler un point de pression de contact appliquée par la surface (500) au corps humain (504) au cours du temps.

10. Procédé selon la revendication 9, dans lequel l'étape de détermination comprend :
la détection, par le dispositif de commande, du nombre d'aimants, le cas échéant, qui sont situés à l'intérieur d'une proximité spécifiée de l'interface de sortie sur la base des sorties produites par le ou les capteurs ;
dans lequel le procédé comprend en outre :
l'identification, par le dispositif de commande, d'un type de tampon d'appareil d'atténuation de pression (100, 200, 300, 400, 502a, 502b, 1306) raccordé fluidiquement à l'interface de sortie sur la base d'un comptage des aimants situés à l'intérieur de la proximité spécifiée ; et
dans lequel le modèle programmable correspond au type de tampon d'atténuation de pression (100, 200, 300, 400, 502a, 502b, 1306).

11. Procédé selon la revendication 9, comprenant en outre :
la réception, par le dispositif de commande, d'une entrée indiquant une demande de lancement d'une procédure de dégonflage ; et
le fait de provoquer, par le dispositif de commande, le dégonflage de fluide à partir d'au moins une chambre du tampon d'atténuation de pression (100, 200, 300, 400, 502a, 502b, 1306) par le biais d'une interface de sortie supplémentaire dans un environnement ambiant ;
dans lequel la demande est :
fournie manuellement par un opérateur par le biais d'une interaction avec un composant d'entrée mécanique du dispositif de commande, ou
fournie automatiquement par le dispositif de commande en réponse à une détermination que la pression d'une chambre du tampon d'atténuation de pression (502a) dépasse un seuil supérieur.

12. Procédé selon la revendication 9, comprenant en outre :
l'acquisition, par le dispositif de commande, de données indiquant la pression de chaque chambre de la pluralité de chambres (508) du tampon d'atténuation de pression (100, 200, 300, 400, 502a, 502b, 1306) ;
l'examen, par le dispositif de commande, des données pour identifier des mouvements, le cas échéant, effectués par le corps humain (504) ;
la détermination, par le dispositif de commande, que le corps humain (504) n'a effectué aucun mouvement pendant une période de temps spécifiée ; et
le fait de provoquer, par le dispositif de commande, la transmission d'une notification à un dispositif électronique,
dans lequel la notification spécifie qu'aucun mouvement n'a été effectué par le corps humain (504) pendant la période de temps spécifiée.

13. Procédé selon la revendication 9, comprenant en outre :
l'acquisition, par le dispositif de commande, de données indiquant la pression de chaque chambre de la pluralité de chambres (508) du tampon d'atténuation de pression (502a) ;
l'examen, par le dispositif de commande, des données pour identifier des mouvements, le cas échéant, effectués par le corps humain (504) ;
la détermination, par le dispositif de commande, que les mouvements effectués par le corps humain (504) correspondent à un modèle spécifié ; et
le fait de provoquer, par le dispositif de commande, la transmission d'une notification à un dispositif électronique,
dans lequel la notification spécifie que les mouvements effectués par le corps humain (504) correspondent au modèle spécifié.

14. Procédé selon la revendication 9, comprenant en outre :
l'acquisition, par le dispositif de commande, de données indiquant la pression de chaque chambre de la pluralité de chambres (508) de l'appareil d'atténuation de pression (100, 200, 300, 400, 502a, 502b, 1306) ; et
l'estimation, par le dispositif de commande, d'une caractéristique du corps humain (504) sur la base des données,
dans lequel la caractéristique est un poids, un rythme cardiaque, ou un rythme respiratoire.
